# EUROPEAN PATENT APPLICATION

(11) **EP 4 382 607 A1**
(43) Date of publication of application: **12.06.2024**
(21) Application number: 22853205.7
(22) Date of filing: 05.08.2022
(51) Int. Cl.: C12N 15/12, A61K 39/00, A61K 39/002, A61K 39/02, A61K 39/12, A61K 39/35, A61K 48/00, A61P 31/00, A61P 31/04, A61P 31/10, A61P 31/12, A61P 33/02, A61P 35/00, A61P 37/04, A61P 37/06, A61P 37/08, A61P 43/00, C07K 14/47, C12N 15/63

(54) **NUCLEIC ACID STRUCTURE UTILIZING SNARE**

(30) Priority: 06.08.2021 JP 2021130022
(71) Applicant: Kao Corporation, Tokyo 103-8210 (JP)
(72) Inventor: SAYAMA, Keimon, Haga-gun, Tochigi 321-3497 (JP); SUGANUMA,Takaya, Haga-gun, Tochigi 321-3497 (JP); HORI, Satoshi, Haga-gun, Tochigi 321-3497 (JP); KUBO, Shun, Haga-gun, Tochigi 321-3497 (JP); YAMAMOTO, Masaki, Haga-gun, Tochigi 321-3497 (JP); FUKAGAWA, Satoko, Haga-gun, Tochigi 321-3497 (JP); ISHIKAWA, Junko, Haga-gun, Tochigi 321-3497 (JP)
(74) Representative: Vossius & Partner Patentanwälte Rechtsanwälte mbB
(86) International application number: PCT/JP2022/030202
(87) International publication number: WO 2023/013787

(57) **Abstract**

Provided is a nucleic acid structure which enhances an allergen-specific immune response. The nucleic acid structure comprises a polynucleotide encoding a SNARE protein and a polynucleotide encoding an allergen.

## Description

### Field of the Invention

The present invention relates to a nucleic acid structure comprising a polynucleotide encoding a SNARE protein and a polynucleotide encoding an allergen.

### Background of the Invention

Vaccine is means for preventing infection and treating a disease by administering an antigen to establish acquired immunity against the antigen. Live vaccine in which an attenuated bacterium or virus is used as it is, inactivated vaccine in which a bacterium or virus is treated with formalin, heat or the like to eliminate its infectious ability, and toxoid vaccine in which a toxin produced by bacteria is separated, purified and treated with formalin or the like to be inactivated have been heretofore administered mainly by subcutaneous injection and intramuscular injection. Besides, mucosal vaccine to be administered to the area of mucosa, nucleic acid vaccine having a nucleic acid as an antigen component, and the like are being developed for the purpose of improving effects, safety and convenience. Among them, nucleic acid vaccine is regarded as a new modality and can be produced cheaply and quickly, and its development proceeds at a rapid rate.

For improving the effect of nucleic acid vaccine, improvement of the immunogenicity of an antigen is one of key challenges, and it is required that an antigen be directed to appropriate tissues or antigen-presenting cells to efficiently trigger an immune response. Patent Literature 1 discloses that the immune response to an antigen was enhanced by using a nucleic acid encoding a fusion protein having an allergen protein (antigen) inserted between an intraorganellar stabilized domain and a transmembrane domain of a lysosomal associated membrane protein (LAMP). On the other hand, it has been reported that addition of an antigen to the LAMP lumen may significantly reduce antigen-specific antibody production (Non Patent Literature 1). Patent Literature 2 discloses that the immunogenicity of an antigen has been increased by using a nucleic acid encoding a fusion molecule containing an antigen, a transmembrane region and a cytoplasmic region of a major histocompatibility complex (MHC) molecular chain. Patent Literature 2 also describes a fusion molecule containing an antigen and a cytoplasmic region of a SNARE (soluble N-ethylmaleimide-sensitive factor attachment protein receptor) protein, but does not either describe an allergen as a specific antigen or show how the immunogenicity of the antigen is changed by the fusion molecule. In an allergy, a pathological condition is formed due mainly to excessive induction of an allergen-specific Th2-type immune response, and therefore, it is desirable that instead of inducing a targeted allergen-specific Th2-type immune response, a Th1-type immune response capable of suppressing the Th2-type immune response be selectively induced in application of nucleic acid vaccine against the allergy. However, an influence on the allergen-specific Th2-type immune response by the use of a nucleic acid encoding the fusion molecule having a transmembrane region and containing an allergen as an antigen is unknown. Thus, an influence exerted by the nucleic acid encoding the fusion molecule with an allergen on the balance between Th1 and Th2 involved in development and suppression of an allergy symptom is unknown. An allergen produced in the body by nucleic acid vaccine may leak into the blood and bind to an allergen-specific IgE antibody present on the surfaces of mast cells and basophils, resulting in occurrence of a type I allergic side effect. To date, a technique for preventing/ameliorating an allergy by a nucleic acid structure has not been established.

The SNARE protein belongs to a family of proteins having a SNARE motif of 20 to 30 kDa. Many of SNARE proteins are linked on a lipid bilayer membrane via a transmembrane domain at the C-terminal, and involved in a process in which a vesicle fuses with a target organelle (Non Patent Literatures 2 and 3). Membrane fusion involving the SNARE protein is mandatory for many important life phenomena indispensable to cell functions in eukaryotic cells, such as endocytosis processes including vesicular transport and formation of an organelle membrane form, and recycling of an extracellular receptor, and exocytosis processes including hormone secretion and release of synaptic neurotransmitters. The molecular mechanisms of membrane fusion by the SNARE protein are considered to be stored in all eukaryotic organisms ranging from single-celled budding yeast to higher animals including humans. The SNARE protein is further classified into four subfamilies of QA-SNARE, QB-SNARE, QC-SNARE and R-SNARE based on the characteristics of amino acid residues of the SNARE motif. It is considered that the SNARE proteins are each localized in specific intracellular membrane fractions (endoplasmic reticula (ER), golgi bodies, endosomes, organelles such as vacuoles and lysosomes, secretory vesicles, cytoplasmic membranes, and the like), and function in the process of membrane fusion in a specific intracellular transport pathway. It is also known that the SNARE protein may be contained in exosomes.

(Patent Literature 1) JP-A-2017-79742
(Patent Literature 2) JP-A-2008-500014
(Non Patent Literature 1) Chen AC, et al. J Immunother Cancer. 2020; 8(1): e000258.
(Non Patent Literature 2) Jahn R, Scheller RH. Nat Rev Mol Cell Biol. 2006; 7(9): 631-643.
(Non Patent Literature 3) Hong W. Biochim Biophys Acta. 2005; 1744(2): 120-144.

### Summary of the Invention

The present invention provides the following 1) to 4)
1) A nucleic acid structure comprising a polynucleotide encoding a SNARE protein and a polynucleotide encoding an allergen.
2) An allergen-specific Th1-type immune response inducing or enhancing agent comprising the nucleic acid structure of 1) as an active component.
3) An allergen-specific cellular immune response inducing or enhancing agent comprising the nucleic acid structure of 1) as an active component.
4) A nucleic acid vaccine comprising the nucleic acid structure of 1) as an active component.

### Brief Description of Drawings

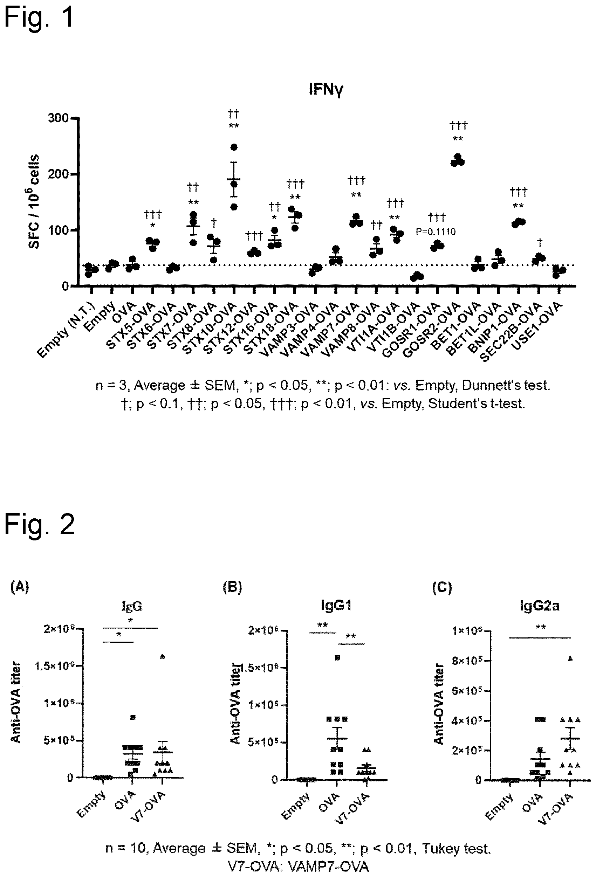

[Fig. 1] Fig. 1 shows the evaluation of the immunogenicity of mRNA encoding SNARE protein-allergen fusion polypeptide. The amount of production of IFNy is shown in terms of the number of production cells (spot forming cell; SFC). "Empty" represents a control in which only a transfection reagent is used, "Empty (N.T.)" represents a control in which restimulation with an allergen is not performed, and "SNARE protein-OVA" represents mRNA encoding a SNARE protein-allergen (OVA) fusion polypeptide.
[Fig. 2] Fig. 2 shows an IgG subclass antibody titer when a plasmid vector encoding a SNARE protein-allergen fusion polypeptide was administered to mice. Fig. 2(A) shows an IgG antibody titer, Fig. 2(B) shows an IgG1 antibody titer, and Fig. 2(C) shows an IgG2a antibody titer. "Empty" represents an empty plasmid vector which does not encode an allergen (OVA), "OVA" represents a plasmid vector encoding OVA, and "V7-OVA" represents a plasmid vector encoding a VAMP7-OVA fusion polypeptide.

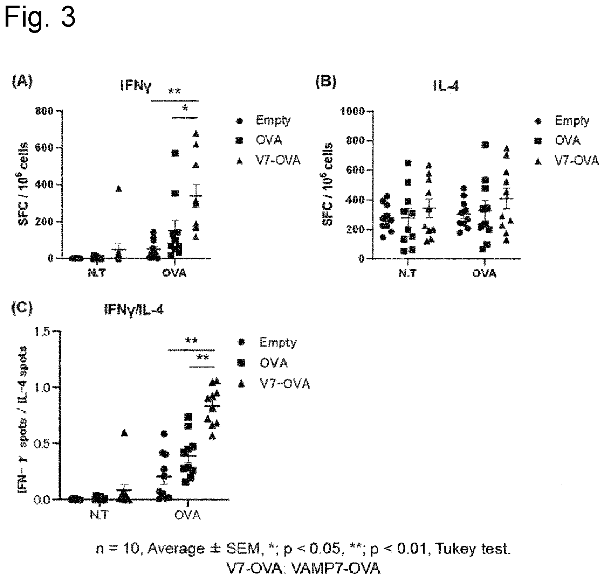

[Fig. 3] Fig. 3 shows the evaluation of the immune response when a plasmid vector encoding a SNARE protein-allergen fusion polypeptide was administered to mice. Fig. 3(A) shows the amount of production of IFNy, and Fig. 3(B) shows the amount of production of IL-4 in terms of SFC. Fig. 3(C) shows a ratio of the amount of production of IFNy to the amount of production of IL-4 (IFNy/IL-4). "Empty" represents an empty plasmid vector which does not encode an allergen (OVA), "OVA" represents a plasmid vector encoding OVA, and "V7-OVA" represents a plasmid vector encoding a VAMP7-OVA fusion polypeptide.

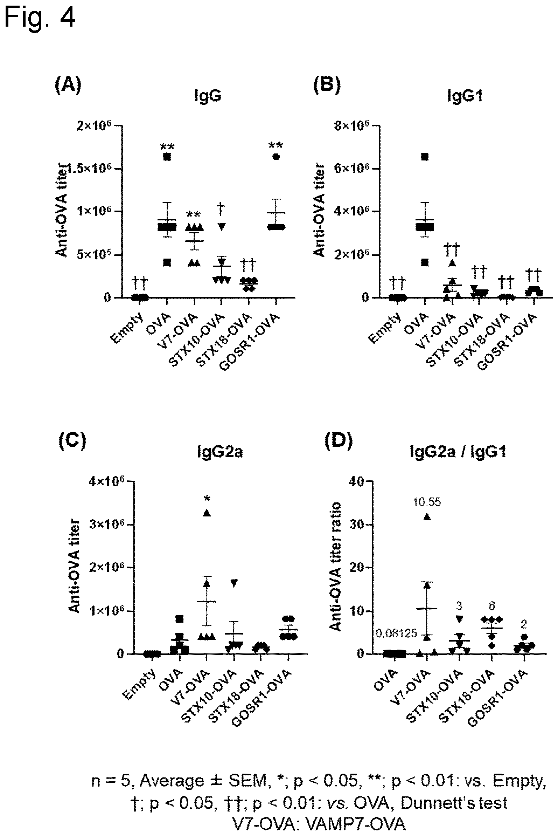

[Fig. 4] Fig. 4 shows an IgG subclass antibody titer when a plasmid vector encoding a SNARE protein-allergen fusion polypeptide was administered to mice. Fig. 4(A) shows an IgG antibody titer, Fig. 4(B) shows an IgG1 antibody titer, Fig. 4(C) shows an IgG2a antibody titer, and Fig. 4(D) shows a ratio of the IgG2a antibody titer to the IgG1 antibody titer (IgG2a/IgG1). "Empty" represents an empty plasmid vector which does not encode an allergen (OVA), "OVA" represents a plasmid vector encoding OVA, and "V7-OVA", "STX10-OVA", "STX18-OVA" and "GOSR1-OVA" represent plasmid vectors encoding VAMP7-OVA, STX10-OVA, STX18-OVA and GOSR1-OVA fusion polypeptides, respectively.

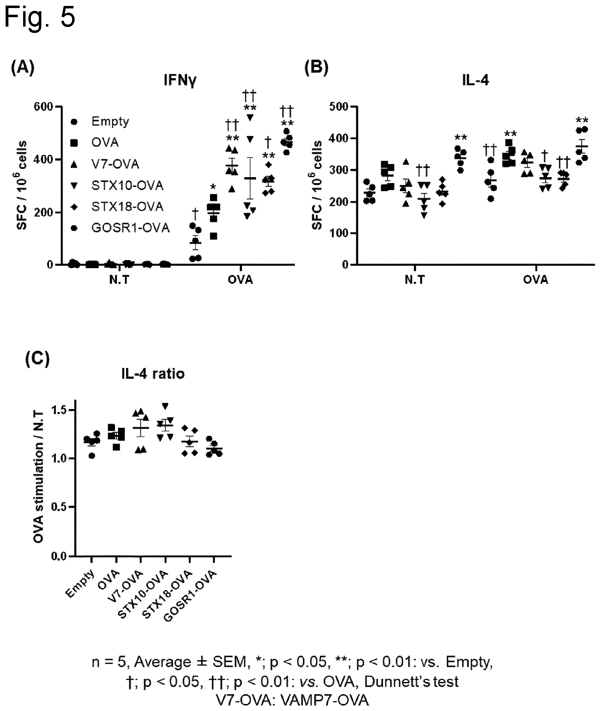

[Fig. 5] Fig. 5 shows the evaluation of the immune response when a plasmid vector encoding a SNARE protein-allergen fusion polypeptide was administered to mice. Fig. 5(A) shows the amount of production of IFNy, and Fig. 5(B) shows the amount of production of IL-4 in terms of SFC. Fig. 5(C) shows a ratio of the amount of production of IL-4 under an allergen-stimulated condition to that under a non-stimulated condition (IL-4 ratio). "Empty" represents an empty plasmid vector which does not encode an allergen (OVA), "OVA" represents a plasmid vector encoding OVA, and "V7-OVA", "STX10-OVA", "STX18-OVA" and "GOSR1-OVA" represent plasmid vectors encoding VAMP7-OVA, STX10-OVA, STX18-OVA and GOSR1-OVA fusion polypeptides, respectively.

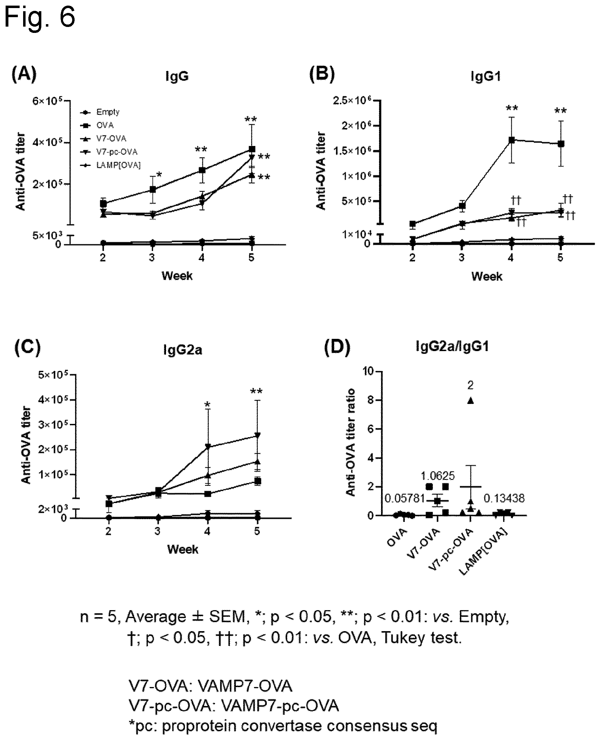

[Fig. 6] Fig. 6 shows an antibody titer over time when a plasmid vector encoding a SNARE protein-allergen fusion polypeptide or a fusion polypeptide containing an allergen in LAMP was administered to mice. Fig. 6(A) represents an IgG antibody titer, Fig. 6(B) represents an IgG1 antibody titer, Fig. 6(C) represents an IgG2a antibody titer, and Fig. 6(D) represents a ratio of the IgG2a antibody titer to the IgG1 antibody titer (IgG2a/IgG1) in the fifth week. "Empty" represents an empty plasmid vector which does not encode an allergen (OVA), "OVA" represents a plasmid vector encoding OVA, "V7-OVA" represents a plasmid vector encoding a VAMP7-OVA fusion polypeptide, "V7-pc-OVA" represents a plasmid vector encoding a VAMP7-OVA fusion polypeptide containing a proprotein convertase recognition sequence, and "LAMP [OVA]" represents a plasmid vector encoding a fusion polypeptide containing OVA within LAMP.

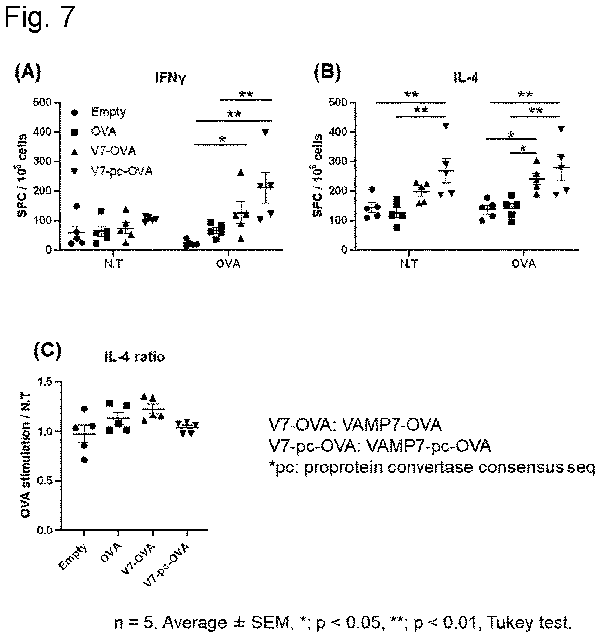

[Fig. 7] Fig. 7 shows the evaluation of the immune response when a plasmid vector encoding a SNARE protein-allergen fusion polypeptide was administered to mice. Fig. 7(A) shows the amount of production of IFNy, and Fig. 7(B) shows the amount of production of IL-4 in terms of SFC. Fig. 7(C) shows a ratio of the amount of production of IL-4 under an allergen-stimulated condition to that under a non-stimulated condition (IL-4 ratio). "Empty" represents an empty plasmid vector which does not encode an allergen (OVA), "OVA" represents a plasmid vector encoding OVA, "V7-OVA" represents a plasmid vector encoding a VAMP7-OVA fusion polypeptide, and "V7-pc-OVA" represents a plasmid vector encoding a VAMP7-OVA fusion polypeptide containing a proprotein convertase recognition sequence.

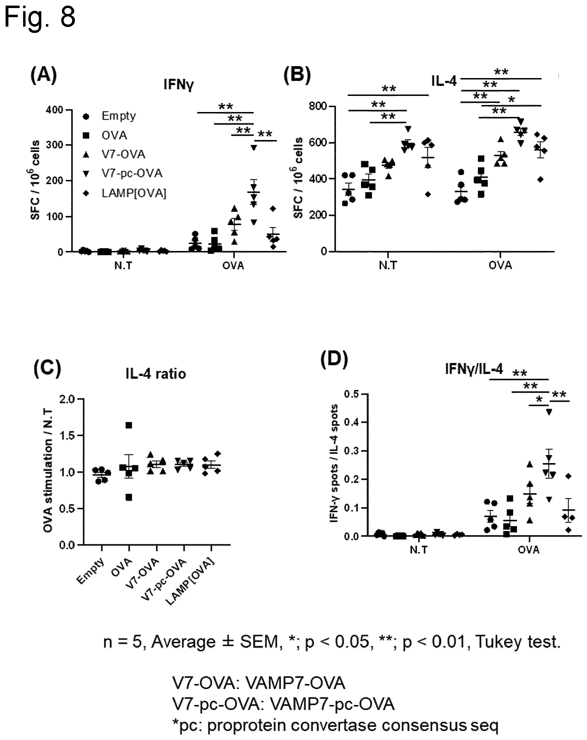

[Fig. 8] Fig. 8 shows the evaluation of the immune response when a plasmid vector encoding a SNARE protein-allergen fusion polypeptide or a fusion polypeptide containing an allergen within LAMP was administered to mice. Fig. 8(A) shows the amount of production of IFNy, and Fig. 8(B) shows the amount of production of IL-4 in terms of SFC. Fig. 8(C) shows a ratio of the amount of production of IL-4 under an allergen-stimulated condition to that under a non-stimulated condition (IL-4 ratio), and Fig. 8(D) shows a ratio of the amount of production of IFN**γ** to the amount of production of IL-4 (IFNy/IL-4). "Empty" represents an empty plasmid vector which does not encode an allergen (OVA), "OVA" represents a plasmid vector encoding OVA, "V7-OVA" represents a plasmid vector encoding a V7-OVA fusion polypeptide, "V7-pc-OVA" represents a plasmid vector encoding a VAMP7-OVA fusion polypeptide containing a proprotein convertase recognition sequence, and "LAMP [OVA]" represents a plasmid vector encoding a fusion polypeptide containing OVA within LAMP.

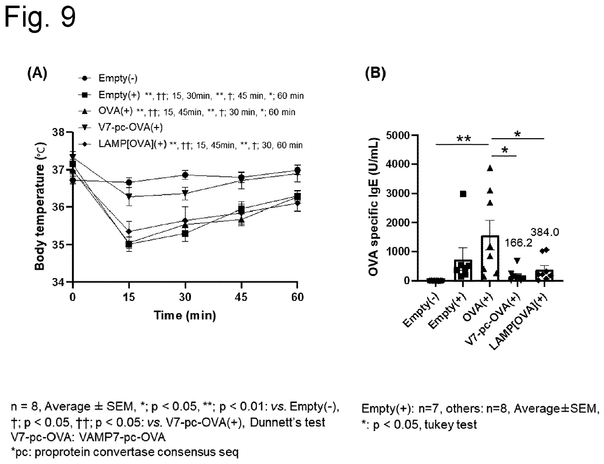

[Fig. 9] Fig. 9 shows the evaluation of the allergic reaction when a plasmid vector encoding a SNARE protein-allergen fusion polypeptide or a fusion polypeptide containing an allergen within LAMP was administered to allergy model mice. Fig. 9(A) shows a change in rectal temperature over time, and Fig. 9(B) shows the amount of production of allergen-specific IgE. "Empty" represents an empty plasmid vector which does not encode an allergen (OVA), "OVA" represents a plasmid vector encoding OVA, "V7-pc-OVA" represents a plasmid vector encoding a VAMP7-proprotein convertase recognition sequence-OVA fusion polypeptide, and "LAMP [OVA]" represents a plasmid vector encoding a fusion polypeptide containing OVA within LAMP. The symbol (-) indicates "non-sensitized", and the symbol (+) indicates "sensitized".

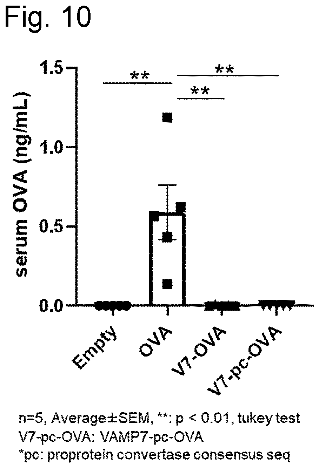

[Fig. 10] Fig. 10 shows the evaluation of the blood concentration of OVA when a plasmid vector encoding a SNARE protein-allergen fusion polypeptide was administered to mice. "Empty" represents an empty plasmid vector which does not encode an allergen (OVA), "OVA" represents a plasmid vector encoding OVA, "V7-OVA" represents a plasmid vector encoding a VAMP7-OVA fusion polypeptide, and "V7-pc-OVA" represents a plasmid vector encoding a VAMP7-proprotein convertase recognition sequence-OVA fusion polypeptide.

### Detailed Description of the Invention

As used herein, the terms "nucleic acid", "nucleotide", "oligonucleotide" and "polynucleotide" are used interchangeably, and each mean DNA or RNA. DNA includes all of cDNA, genomic DNA and synthetic DNA, and RNA includes all of total RNA, mRNA, rRNA, tRNA, noncoding RNA and synthetic RNA. Among them, mRNA is synthesized by an *in vitro* transcription reaction, followed by addition of 5'-cap (methylated guanosine) by a capping enzyme and addition of poly A by a poly (A) polymerase for starting a translation reaction *in vivo,* stabilization of mRNA, suppression of degradation and the like. The poly A sequence may be incorporated in template DNA for use in the *in vitro* transcription reaction. In this case, mRNA also includes mRNA to which a cap structure or poly A is added, and RNA in which some bases are modified (for example, uridine is replaced with pseudouridine or 1-methylpseudouridine).

As used herein, the term "gene" means DNA which includes double-stranded DNA including genomic DNA, single-stranded DNA including cDNA (plus strand), single-stranded DNA having a sequence complementary to the plus strand (complementary strand), and fragments thereof, where the information of sequences of bases forming DNA includes some biological information.

The "gene" includes not only "genes" represented by a specific nucleotide sequence, but also nucleic acids encoding congeners (i.e., homologs or orthologs), variants such as genetic polymorphisms, and derivatives thereof.

The names and gene IDs of genes disclosed herein conform to the official symbols and gene IDs specified by NCBI ([www.ncbi.nlm.nih.gov/]).

As used herein, the terms "peptide", "polypeptide" and "protein" are used interchangeably.

As used herein, the term "amino acid residue" means 20 amino acid residues forming proteins, that is, alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysin (Lys or K), methionine (Met or M), phenylalanine (Phe or F), proline (Pro or P), serine (Ser or S), threonine (Thr or T), tryptophan (Trp or W), tyrosine (Tyr or Y) and valine (Val or V).

As used herein, the identity between nucleotide sequences or amino acid sequences is calculated by a Lipman-Pearson method (Science, 1985, 227: 1435-1441). Specifically, the identity can be calculated by performing analysis using the homology analysis (search homology) program of genetic information processing software Genetyx-Win, where the unit size to compare (ktup) is set to 2.

As used herein, the term "identity of at least 80%" concerning nucleotide sequences or amino acid sequences refers to an identity of 80% or more, preferably 85% or more, more preferably 90% or more, further more preferably 95% or more, even more preferably 98% or more, even more preferably 99% or more.

As used herein, unless otherwise specified, the term "one or several" used in the context of deletion, substitution, addition or insertion of nucleotides in a nucleotide sequence may mean preferably from 1 to 15, more preferably from 1 to 9, further more preferably from 1 to 6. As used herein, unless otherwise specified, the term "one or several" used in the context of deletion, substitution, addition or insertion of amino acid residues may mean preferably from 1 to 5, more preferably from 1 to 3, further more preferably from 1 or 2. As used herein, the "addition" of nucleotides or amino acid residues includes addition of nucleotides or amino acid residues to one end or both ends of a sequence.

Examples of the "stringent conditions" as used herein include conditions of the Southern hybridization method described in Molecular Cloning-A LABORATORY MANUAL THIRD EDITION (Joseph Sambrook, David W. Russell., Cold Spring Harbor Laboratory Press, 2001), for example, a condition of being held together with a probe at 42°C for 8 to 16 hours in a solution containing 6 × SSC (composition of 1 × SSC: 0.15 M sodium chloride, 0.015 M sodium citrate, pH 7.0), 0.5% SDS, 5 × Denhardt and 100 mg/mL herring sperm DNA, thereby being hybridized.

As used herein, the term "fragment" of a polynucleotide means a partial polynucleotide of the polynucleotide. The length of the partial polynucleotide is not limited as long as it encodes a polypeptide functionally equivalent to a polypeptide encoded by the full-length polynucleotide. For example, the partial polynucleotide may mean a polynucleotide consisting of consecutive nucleotides and having a length of preferably 30% or more, more preferably 40% or more, further more preferably 50% or more, and preferably 90% or less, more preferably 80% or less, further more preferably 70% or less, even more preferably 60% or less with respect to the full-length polynucleotide. As used herein, the term "fragment" of a polypeptide means a partial polypeptide of the polypeptide. The length of the partial polypeptide is not limited. For example, the partial polypeptide may mean a polypeptide consisting of consecutive amino acid residues and having a length of preferably 30% or more, more preferably 40% or more, further more preferably 50% or more, and preferably 90% or less, more preferably 80% or less, further more preferably 70% or less, even more preferably 60% or less with respect to the full-length polypeptide.

As used herein, the term "control region" refers to a region having a function of controlling expression of a gene (for example, a region encoding a protein) downstream of the region. More specifically, the "control region" can be defined as a region which is present upstream of a coding region in the gene, and has a function of interacting with RNA polymerases to control transcription of the coding region. The control region includes a transcription start control region and/or translation start control region, or a region extending from the transcription start control region to the translation start control region. The transcription start control region includes a promoter and a transcription start point, and the translation start control region is a site which is recognized together with a start codon by ribosomes, and corresponds to a Kozak sequence necessary for the start of translation.

As used herein, the phrase "operable linkage" of a control region and a polynucleotide of a gene (for example, a polynucleotide encoding a protein)" means that the gene and the control region are linked such that the gene can be expressed under the control of the control region. The procedure for "operable linkage" of a gene and a control region is well known to those skilled in the art.

As used herein, the phrase "expressible linkage" of a polynucleotide of a first gene (for example, a polynucleotide encoding a protein) and a polynucleotide of a second gene (for example, a polynucleotide encoding a protein)" means that when a first gene and a second gene are inserted into an appropriate expression vector and the expression vector is introduced into appropriate cells, the polynucleotides are linked such that a protein encoded by the first gene and a protein encoded by the second gene are produced as a fusion protein. Here, the term "linkage" is a concept including a case where the first gene and the second gene are directly bonded to each other, and a case where the first gene and the second gene are bound to each other via another nucleotide sequence. The procedure for "expressible linkage" of the first gene and the second gene is well known to those skilled in the art.

As used herein, the terms "upstream" and "downstream" concerning a gene or a nucleotide sequence of the gene refer to the upstream and the downstream, respectively, in the transcriptional direction of the gene. For example, the terms "upstream sequence" and "downstream sequence" of a gene refer to sequences located 5' and 3' of the gene, respectively, in a DNA sense strand.

As used herein, the term "antigen" means a molecule which causes an immune response such as production of an antibody or cellular immunity in living organisms. Specifically, the term "antigen" refers to an allergen. As used herein, the term "immunogenicity" means a property of an antigen to induce production of an antibody and cellular immunity.

As used herein, the term "nucleic acid vaccine" refers to vaccine that is administered as a polynucleotide (DNA or RNA) encoding an antigen to a living organism to induce immunity. The nucleic acid vaccine includes DNA vaccine, mRNA vaccine and virus vector vaccine, all of which are considered to induce both humoral immunity and cellular immunity. The DNA vaccine contains a plasmid encoding an antigen. A plasmid administered to a living organism is incorporated into cells and transcribed into mRNA in the nucleus, and the mRNA is translated into an antigenic protein in the cytoplasm, so that an antigen-specific immune response is induced. The mRNA vaccine contains mRNA encoding an antigen. mRNA administered to a living organism is incorporated into cells, and the mRNA is translated into an antigenic protein in the cytoplasm, so that an antigen-specific immune response is induced. The virus vector vaccine contains a non-pathogenic or attenuated virus vector into which a polynucleotide encoding an antigen is incorporated. A virus administered to a living organism enters cells, and causes the cells to synthesize an antigenic protein, so that an antigen-specific immune response is induced.

As used herein, the term "cellular immunity" refers to immune responses using cytotoxic T cells, macrophages, NK cells and the like as an effector, among acquired immune responses which serve to eliminate foreign matters such as pathogens entering a living organism, virus-infected cells and cancer cells. As used herein, the term "humoral immunity" refers to immune responses using an antibody as an effector, among acquired immune responses.

As used herein, the term "Th1-type immune response" refers to immune responses which are promoted by Th1 cells, among subsets of helper T cells. Th1 cells produce mainly IFNy as cytokines, target mainly M1 macrophages, cytotoxic T cells and NK cells, and induce mainly cellular immunity. It is known that Th1 cells activate B cells to induce production of IgG2a. As used herein, the term "Rh2-type immune response" refers to immune responses which are promoted by Th2 cells, among subsets of helper T cells. Th2 cells produce mainly IL-4, IL-5 and IL-13 as cytokines, target mainly mast cells, M2 macrophages, eosinophils and basophils, and induce mainly humoral immunity. It is known that Th2 cells activate B cells to induce production of IgG1.

The present invention relates to provision of a nucleic acid structure which enhances an allergen-specific immune response.

The present inventors found that by using a nucleic acid structure containing a polynucleotide encoding a SNARE protein and a polynucleotide encoding an allergen, an allergen-specific Th1-type immune response can be induced or enhanced and an allergen-specific cellular immune response can be induced or enhanced as compared to a nucleic acid structure containing only a polynucleotide encoding an allergen.

According to the nucleic acid structure of the present invention, an allergen-specific Th1-type immune response can be induced or enhanced, and an allergen-specific cellular immune response can be induced or enhanced. Such a nucleic acid structure is useful as nucleic acid vaccine.

The present invention provides a nucleic acid structure containing a polynucleotide encoding a SNARE protein and a polynucleotide encoding an allergen.

In the present invention, the SNARE protein is a protein belonging to a family of proteins having a SNARE motif. The SNARE protein is classified into four subfamilies of QA-SNARE, QB-SNARE, QC-SNARE and R-SNARE based on the characteristics of amino acid residues of the SNARE motif, and the SNARE protein for use in the present invention may belong to any of the subfamilies. The SNARE protein is preferably one that has a transmembrane domain and is localized in intracellular vesicles. Specific examples thereof include, but are not limited to, VAMP7, GOSR2, STX10, STX18, BNIP1, STX7, VTI1A, STX16, STX5, GOSR1, STX8, STX12, VAMP8, SEC22B, STX6, VAMP3, VAMP4, VTI1B, BET1, BET1L, and USE1. From the viewpoint of induction or enhancement of immunity, the SNARE protein is preferably any one selected from the group consisting of VAMP7, GOSR2, STX10, STX18, BNIP1, STX7, VTI1A, STX16, STX5, GOSR1, STX8, STX12, VAMP8 and SEC22B, more preferably any one selected from the group consisting of VAMP7, GOSR2, STX10, STX18, BNIP1, STX7, VTI1A, STX16, STX5 and GOSR1, further more preferably any one selected from the group consisting of VAMP7, GOSR2, STX10, STX18, BNIP1, STX7, VTI1A and GOSR1, further more preferably any one selected from the group consisting of VAMP7, GOSR2, STX10 and GOSR1, further more preferably VAMP7.

VAMP7 (vesicle-associated membrane protein 7) is one of SNARE proteins, has a transmembrane domain, and is thought to be localized in late endosomes, lysosomes and cell membranes. In a preferred example, VAMP7 is mammal VAMP7. In a more preferred example, VAMP7 is human VAMP7, and is a protein which consists of the amino acid sequence of SEQ ID NO: 10, and is encoded by a gene consisting of the nucleotide sequence of SEQ ID NO: 1 (gene ID: 6845). The term VAMP7 for use in the present invention includes VAMP7, and polypeptides functionally equivalent to VAMP7.

GOSR2 (golgi SNAP receptor complex member 2) is one of SNARE proteins, has a transmembrane domain, and is thought to be localized in endoplasmic reticulum-golgi intermediate compartments and golgi bodies. In a preferred example, GOSR2 is mammal GOSR2. In more preferred example, GOSR2 is human GOSR2, and is a protein which consists of the amino acid sequence of SEQ ID NO: 11, and is encoded by a gene consisting of the nucleotide sequence of SEQ ID NO: 2 (gene ID: 9570). The term GOSR2 for use in the present invention includes GOSR2, and polypeptides functionally equivalent to GOSR2.

STX (syntaxin) 10 is one of SNARE proteins, has a transmembrane domain, and is thought to be localized in trans-golgi networks. In a preferred example, STX10 is mammal STX10. In a more preferred example, STX10 is human STX10, and is a protein which consists of the amino acid sequence of SEQ ID NO: 12, and is encoded by a gene consisting of the nucleotide sequence of SEQ ID NO: 3 (gene ID: 8677). The term STX10 for use in the present invention includes STX10, and polypeptides functionally equivalent to STX10.

STX18 is one of SNARE proteins, has a transmembrane domain, and is thought to be localized in endoplasmic reticula. In a preferred example, STX18 is mammal STX18. In a more preferred example, STX18 is human STX18, and is a protein which consists of the amino acid sequence of SEQ ID NO: 13, and is encoded by a gene consisting of the nucleotide sequence of SEQ ID NO: 4 (gene ID: 53407). The term STX18 for use in the present invention includes STX18, and polypeptides functionally equivalent to STX18.

BNIP1 (BCL2 interacting protein 1) is one of SNARE proteins, has a transmembrane domain, and is thought to be localized in endoplasmic reticula. In a preferred example, BNIP1 is mammal BNIP1. In a more preferred example, BNIP1 is human BNIP1, and is a protein which consists of the amino acid sequence of SEQ ID NO: 14, and is encoded by a gene consisting of the nucleotide sequence of SEQ ID NO: 5 (gene ID: 662). The term BNIP1 for use in the present invention includes BNIP1, and polypeptides functionally equivalent to BNIP1.

STX7 is one of SNARE proteins, has a transmembrane domain, and is thought to be localized in early endosomes and late endosomes. In a preferred example, STX7 is mammal STX7. In a more preferred example, STX7 is human STX7, and is a protein which consists of the amino acid sequence of SEQ ID NO: 15, and is encoded by a gene consisting of the nucleotide sequence of SEQ ID NO: 6 (gene ID: 8417). The term STX7 for use in the present invention includes STX7, and polypeptides functionally equivalent to STX7.

VTI1A (vesicle transport through interaction with t-SNAREs 1A) is one of SNARE proteins, has a transmembrane domain, and is thought to be localized in trans-golgi networks. In a preferred example, VTI1A is mammal VTI1A. In a more preferred example, VTI1A is human VTI1A, and is a protein which consists of the amino acid sequence of SEQ ID NO: 16, and is encoded by a gene consisting of the nucleotide sequence of SEQ ID NO: 7 (gene ID: 143187). The term VTI1A for use in the present invention includes VTI1A, and polypeptides functionally equivalent to VTI1A.

STX16 is one of SNARE proteins, has a transmembrane domain, and is thought to be localized in trans-golgi networks. In a preferred example, STX16 is mammal STX16. In a more preferred example, STX16 is human STX16, and is a protein which consists of the amino acid sequence of SEQ ID NO: 17, and is encoded by a gene consisting of the nucleotide sequence of SEQ ID NO: 8 (gene ID: 8675). The term STX16 for use in the present invention includes STX16, and polypeptides functionally equivalent to STX16.

STX5 is one of SNARE proteins, has a transmembrane domain, and is thought to be localized in golgi bodies. In a preferred example, STX5 is mammal STX5. In a more preferred example, STX5 is human STX5, and is a protein which consists of the amino acid sequence of SEQ ID NO: 18, and is encoded by a gene consisting of the nucleotide sequence of SEQ ID NO: 9 (gene ID: 6811). The term STX5 for use in the present invention includes STX5, and polypeptides functionally equivalent to STX5.

GOSR1 is one of SNARE proteins, has a transmembrane domain, and is thought to be localized in golgi bodies and trans-golgi networks. In a preferred example, GOSR1 is mammal GOSR1. In a more preferred example, GOSR1 is human GOSR1, and is a protein which consists of the amino acid sequence of SEQ ID NO: 83, and is encoded by a gene consisting of the nucleotide sequence of SEQ ID NO: 71 (gene ID: 9527). The term GOSR1 for use in the present invention includes GOSR1, and polypeptides functionally equivalent to GOSR1.

STX8 is one of SNARE proteins, has a transmembrane domain, and is thought to be localized in early endosomes, late endosomes and cell membranes. In a preferred example, STX8 is mammal STX8. In a more preferred example, STX8 is human STX8, and is a protein which consists of the amino acid sequence of SEQ ID NO: 84, and is encoded by a gene consisting of the nucleotide sequence of SEQ ID NO: 72 (gene ID: 9482). The term STX8 for use in the present invention includes STX8, and polypeptides functionally equivalent to STX8.

STX12 is one of SNARE proteins, has a transmembrane domain, and is thought to be localized in golgi bodies, early endosomes and recycling endosomes. In a preferred example, STX12 is mammal STX12. In a more preferred example, STX12 is human STX12, and is a protein which consists of the amino acid sequence of SEQ ID NO: 85, and is encoded by a gene consisting of the nucleotide sequence of SEQ ID NO: 73 (gene ID: 23673). The term STX12 for use in the present invention includes STX12, and polypeptides functionally equivalent to STX12.

VAMP8 is one of SNARE proteins, has a transmembrane domain, and is thought to be localized in lysosomes, early endosomes, late endosomes and cell membranes. In a preferred example, VAMP8 is mammal VAMP8. In a more preferred example, VAMP8 is human VAMP8, and is a protein which consists of the amino acid sequence of SEQ ID NO: 86, and is encoded by a gene consisting of the nucleotide sequence of SEQ ID NO: 74 (gene ID: 8673). The term VAMP8 for use in the present invention includes VAMP8, and polypeptides functionally equivalent to VAMP8.

SEC22B (SEC22 homolog B, vesicle trafficking protein) is one of SNARE proteins, has a transmembrane domain, and is thought to be localized in endoplasmic reticula, endoplasmic reticulum-golgi intermediate compartments, golgi bodies, cis-golgi networks and trans-golgi networks. In a preferred example, SEC22B is mammal SEC22B. In a more preferred example, SEC22B is human SEC22B, and is a protein which consists of the amino acid sequence of SEQ ID NO: 87, and is encoded by a gene consisting of the nucleotide sequence of SEQ ID NO: 75 (gene ID: 9554). The term SEC22B for use in the present invention includes SEC22B, and polypeptides functionally equivalent to SEC22B.

STX6 is one of SNARE proteins, has a transmembrane domain, and is thought to be localized in golgi bodies. In a preferred example, STX6 is mammal STX6. In a more preferred example, STX6 is human STX6, and is a protein which consists of the amino acid sequence of SEQ ID NO: 88, and is encoded by a gene consisting of the nucleotide sequence of SEQ ID NO: 76 (gene ID: 10228). The term STX6 for use in the present invention includes STX6, and polypeptides functionally equivalent to STX6.

VAMP3 (vesicle-associated membrane protein 3) is one of SNARE proteins, has a transmembrane domain, and is thought to be localized in cell membranes and golgi bodies. In a preferred example, VAMP3 is mammal VAMP3. In a more preferred example, VAMP3 is human VAMP3, and is a protein which consists of the amino acid sequence of SEQ ID NO: 89, and is encoded by a gene consisting of the nucleotide sequence of SEQ ID NO: 77 (gene ID: 9341). The term VAMP3 for use in the present invention includes VAMP3, and polypeptides functionally equivalent to VAMP3.

VAMP4 (vesicle-associated membrane protein 4) is one of SNARE proteins, has a transmembrane domain, and is thought to be localized in golgi bodies and trans-golgi networks. In a preferred example, VAMP4 is mammal VAMP4. In a more preferred example, VAMP4 is human VAMP4, and is a protein which consists of the amino acid sequence of SEQ ID NO: 90, and is encoded by a gene consisting of the nucleotide sequence of SEQ ID NO: 78 (gene ID: 8674). The term VAMP4 for use in the present invention includes VAMP4, and polypeptides functionally equivalent to VAMP4.

VTI1B (vesicle transport through interaction with t-SNAREs homolog 1B) is one of SNARE proteins, has a transmembrane domain, and is thought to be localized in early endosomes, late endosomes, lysosomes and recycling endosomes. In a preferred example, VTI1B is mammal VTI1B. In a more preferred example, VTI1B is human VTI1B, and is a protein which consists of the amino acid sequence of SEQ ID NO: 91, and is encoded by a gene consisting of the nucleotide sequence of SEQ ID NO: 79 (gene ID: 10490). The term VTI1B for use in the present invention includes VTI1B, and polypeptides functionally equivalent to VTI1B.

BET1 (Bet1 golgi vesicular membrane trafficking protein) is one of SNARE proteins, has a transmembrane domain, and is thought to be localized in endoplasmic reticulum, golgi bodies and cis-golgi networks. In a preferred example, BET1 is mammal BET1. In a more preferred example, BET1 is human BET1, and is a protein which consists of the amino acid sequence of SEQ ID NO: 92, and is encoded by a gene consisting of the nucleotide sequence of SEQ ID NO: 80 (gene ID: 10282). The term BET1 for use in the present invention includes BET1, and polypeptides functionally equivalent to BET1.

BET1L (Bet1 golgi vesicular membrane trafficking protein like) is one of SNARE proteins, has a transmembrane domain, and is thought to be localized in golgi bodies and trans-golgi networks. In a preferred example, BET1L is mammal BET1L. In a more preferred example, BET1L is human BET1L, and is a protein which consists of the amino acid sequence of SEQ ID NO: 93, and is encoded by a gene consisting of the nucleotide sequence of SEQ ID NO: 81 (gene ID: 51272). The term BET1L for use in the present invention includes BET1L, and polypeptides functionally equivalent to BET1L.

USE1 (unconventional SNARE in the ER 1) is one of SNARE proteins, has a transmembrane domain, and is thought to be localized in endoplasmic reticula. In a preferred example, USE1 is mammal USE1. In a more preferred example, USE1 is human USE1, and is a protein which consists of the amino acid sequence of SEQ ID NO: 94, and is encoded by a gene consisting of the nucleotide sequence of SEQ ID NO: 82 (gene ID: 55850). The term USE1 for use in the present invention includes USE1, and polypeptides functionally equivalent to USE1.

In the present invention, the phrase "polypeptide functionally equivalent to a SNARE protein" refers to a polypeptide equivalent in biological activity to the SNARE protein. Examples of the polypeptide include polypeptides which have a transmembrane domain, and can be localized in intracellular vesicles (preferably intracellular vesicles in which a corresponding SNARE protein is localized) or exosomes. Specific examples include the following polypeptides (b) to (e) or (b), (c) and (e).

Specific examples of VAMP7 include the following:
(a) a polypeptide consisting of the amino acid sequence of SEQ ID NO: 10;
(b) a polypeptide which consists of an amino acid sequence having an identity of at least 80% with the amino acid sequence of SEQ ID NO: 10, has a transmembrane domain, and can be localized in intracellular vesicles (for example, late endosomes or lysosomes) or exosomes;
(c) a polypeptide which consists of an amino acid sequence obtained by deletion, substitution, addition or insertion of one or several amino acids with respect to the amino acid sequence of SEQ ID NO: 10, has a transmembrane domain, and can be localized in intracellular vesicles (for example, late endosomes or lysosomes) or exosomes;
(d) a polypeptide which consists of an amino acid sequence encoded by a splicing variant of a polynucleotide consisting of the nucleotide sequence of SEQ ID NO: 1, has a transmembrane domain, and can be localized in intracellular vesicles (for example, late endosomes or lysosomes) or exosomes; and
(e) a polypeptide which is a fragment of any of the polypeptides (a) to (d), has a transmembrane domain, and can be localized in intracellular vesicles (for example, late endosomes or lysosomes) or exosomes.

Examples of the splicing variant of the VAMP7 gene consisting of the nucleotide sequence of SEQ ID NO: 1 include variants which are deposited as NM_001185183.2 and NM_001145149.3 and encode proteins deposited, respectively, as NP_001172112.1 and NP_001138621.1 in RefSeq (Reference Sequence) of NCBI.

Specific examples of GOSR2 include the following:
(a) a polypeptide consisting of the amino acid sequence of SEQ ID NO: 11;
(b) a polypeptide which consists of an amino acid sequence having an identity of at least 80% with the amino acid sequence of SEQ ID NO: 11, has a transmembrane domain, and can be localized in intracellular vesicles (for example, endoplasmic reticulum-golgi intermediate compartments or golgi bodies) or exosomes;
(c) a polypeptide which consists of an amino acid sequence obtained by deletion, substitution, addition or insertion of one or several amino acids with respect to the amino acid sequence of SEQ ID NO: 11, has a transmembrane domain, and can be localized in intracellular vesicles (for example, endoplasmic reticulum-golgi intermediate compartments or golgi bodies) or exosomes;
(d) a polypeptide which consists of an amino acid sequence encoded by a splicing variant of a polynucleotide consisting of the nucleotide sequence of SEQ ID NO: 2, has a transmembrane domain, and can be localized in intracellular vesicles (for example, endoplasmic reticulum-golgi intermediate compartments or golgi bodies) or exosomes; and
(e) a polypeptide which is a fragment of any of the polypeptides (a) to (d), has a transmembrane domain, and can be localized in intracellular vesicles (for example, endoplasmic reticulum-golgi intermediate compartments or golgi bodies) or exosomes.

Examples of the splicing variant of the GOSR2 gene consisting of the nucleotide sequence of SEQ ID NO: 2 include variants which are deposited as NM_054022.4, NM_004287.5, NM_001353114.2, NM_001012511.3, NM_001363851.2, NM_001330252.2, NM_001353116.2, NM_001353115.2 and NM_001321134.2 and encode proteins deposited, respectively, as NP_473363.1, NP_004278.2, NP_001340043.1, NP_001012529.1, NP_001350780.1, NP_001317181.1, NP_001340045.1, NP_001340044.1 and NP_001308063.1 in RefSeq of NCBI.

Specific examples of STX10 include the following:
(a) a polypeptide consisting of the amino acid sequence of SEQ ID NO: 12;
(b) a polypeptide which consists of an amino acid sequence having an identity of at least 80% with the amino acid sequence of SEQ ID NO: 12, has a transmembrane domain, and can be localized in intracellular vesicles (for example, trans-golgi networks) or exosomes;
(c) a polypeptide which consists of an amino acid sequence obtained by deletion, substitution, addition or insertion of one or several amino acids with respect to the amino acid sequence of SEQ ID NO: 12, has a transmembrane domain, and can be localized in intracellular vesicles (for example, trans-golgi networks) or exosomes;
(d) a polypeptide which consists of an amino acid sequence encoded by a splicing variant of a polynucleotide consisting of the nucleotide sequence of SEQ ID NO: 3, has a transmembrane domain, and can be localized in intracellular vesicles (for example, trans-golgi networks) or exosomes; and
(e) a polypeptide which is a fragment of any of the polypeptides (a) to (d), has a transmembrane domain, and can be localized in intracellular vesicles (for example, trans-golgi networks) or exosomes.

Examples of the splicing variant of the STX10 gene consisting of the nucleotide sequence of SEQ ID NO: 3 include variants which are deposited as NM_001271610.2, NM_1271609.2 and NM_001271611.2 and encode proteins deposited, respectively, as NP_001258539.1, NP_001258538.1 and NP_001258540.1 in RefSeq of NCBI.

Specific examples of STX18 include the following:
(a) a polypeptide consisting of the amino acid sequence of SEQ ID NO: 13;
(b) a polypeptide which consists of an amino acid sequence having an identity of at least 80% with the amino acid sequence of SEQ ID NO: 13, has a transmembrane domain, and can be localized in intracellular vesicles (for example, endoplasmic reticula) or exosomes;
(c) a polypeptide which consists of an amino acid sequence obtained by deletion, substitution, addition or insertion of one or several amino acids with respect to the amino acid sequence of SEQ ID NO: 13, has a transmembrane domain, and can be localized in intracellular vesicles (for example, endoplasmic reticula) or exosomes;
(d) a polypeptide which consists of an amino acid sequence encoded by a splicing variant of a polynucleotide consisting of the nucleotide sequence of SEQ ID NO: 4, has a transmembrane domain, and can be localized in intracellular vesicles (for example, endoplasmic reticula) or exosomes; and
(e) a polypeptide which is a fragment of any of the polypeptides (a) to (d), has a transmembrane domain, and can be localized in intracellular vesicles (for example, endoplasmic reticula) or exosomes.

Examples of the splicing variant of the STX18 gene consisting of the nucleotide sequence of SEQ ID NO: 4 include variants which are deposited as NM_001346281.2, NM_001346282.2 and NM_001346300.2 and encode proteins deposited, respectively, as NP_001333210.1, NP_001333211.1 and NP_001333229.1 in RefSeq of NCBI.

Specific examples of BNIP1 include the following:
(a) a polypeptide consisting of the amino acid sequence of SEQ ID NO: 14;
(b) a polypeptide which consists of an amino acid sequence having an identity of at least 80% with the amino acid sequence of SEQ ID NO: 14, has a transmembrane domain, and can be localized in intracellular vesicles (for example, endoplasmic reticula) or exosomes;
(c) a polypeptide which consists of an amino acid sequence obtained by deletion, substitution, addition or insertion of one or several amino acids with respect to the amino acid sequence of SEQ ID NO: 14, has a transmembrane domain, and can be localized in intracellular vesicles (for example, endoplasmic reticula) or exosomes;
(d) a polypeptide which consists of an amino acid sequence encoded by a splicing variant of a polynucleotide consisting of the nucleotide sequence of SEQ ID NO: 5, has a transmembrane domain, and can be localized in intracellular vesicles (for example, endoplasmic reticula) or exosomes; and
(e) a polypeptide which is a fragment of any of the polypeptides (a) to (d), has a transmembrane domain, and can be localized in intracellular vesicles (for example, endoplasmic reticula) or exosomes.

Examples of the splicing variant of the BNIP1 gene consisting of the nucleotide sequence of SEQ ID NO: 5 include variants which are deposited as NM_013980.3, NM_001205.3 and NM_013978.3 and encode proteins deposited, respectively, as NP_053583.2, NP_001196.2 and NP_053581.2 in RefSeq of NCBI.

Specific examples of STX7 include the following:
(a) a polypeptide consisting of the amino acid sequence of SEQ ID NO: 15;
(b) a polypeptide which consists of an amino acid sequence having an identity of at least 80% with the amino acid sequence of SEQ ID NO: 15, has a transmembrane domain, and can be localized in intracellular vesicles (for example, early endosomes or late endosomes) or exosomes;
(c) a polypeptide which consists of an amino acid sequence obtained by deletion, substitution, addition or insertion of one or several amino acids with respect to the amino acid sequence of SEQ ID NO: 15, has a transmembrane domain, and can be localized in intracellular vesicles (for example, early endosomes or late endosomes) or exosomes;
(d) a polypeptide which consists of an amino acid sequence encoded by a splicing variant of a polynucleotide consisting of the nucleotide sequence of SEQ ID NO: 6, has a transmembrane domain, and can be localized in intracellular vesicles (for example, early endosomes or late endosomes) or exosomes; and
(e) a polypeptide which is a fragment of any of the polypeptides (a) to (d), has a transmembrane domain, and can be localized in intracellular vesicles (for example, early endosomes or late endosomes) or exosomes.

Examples of the splicing variant of the STX7 gene consisting of the nucleotide sequence of SEQ ID NO: 6 include variants which are deposited as NM_003569.3, NM_001326578.2, NM_001326579.2 and NM_001326580.2 and encode proteins deposited, respectively, as NP_003560.2, NP_001313507.1, NP_001313508.1 and NP_001313509.1 in RefSeq of NCBI.

Specific examples of VAMP7 include the following:
(a) a polypeptide consisting of the amino acid sequence of SEQ ID NO: 16;
(b) a polypeptide which consists of an amino acid sequence having an identity of at least 80% with the amino acid sequence of SEQ ID NO: 16, has a transmembrane domain, and can be localized in intracellular vesicles (for example, trans-golgi networks) or exosomes;
(c) a polypeptide which consists of an amino acid sequence obtained by deletion, substitution, addition or insertion of one or several amino acids with respect to the amino acid sequence of SEQ ID NO: 16, has a transmembrane domain, and can be localized in intracellular vesicles (for example, trans-golgi networks) or exosomes;
(d) a polypeptide which consists of an amino acid sequence encoded by a splicing variant of a polynucleotide consisting of the nucleotide sequence of SEQ ID NO: 7, has a transmembrane domain, and can be localized in intracellular vesicles (for example, trans-golgi networks) or exosomes; and
(e) a polypeptide which is a fragment of any of the polypeptides (a) to (d), has a transmembrane domain, and can be localized in intracellular vesicles (for example, trans-golgi compartments) or exosomes.

Examples of the splicing variant of the VTI1A gene consisting of the nucleotide sequence of SEQ ID NO: 7 include variants which are deposited as NM_145206.4, NM_001365711.1, NM_001365710.2, NM_001365712.1, NM_001365713.1, NM_001365714.1 and NM_001318205.2 and encode proteins deposited, respectively, as NP_660207.2, NP_001352640.1, NP_001352639.1, NP_001352641.1, NP_001352642.1, NP_001352643.1 and NP_001305134.1 in RefSeq of NCBI.

Specific examples of STX16 include the following:
(a) a polypeptide consisting of the amino acid sequence of SEQ ID NO: 17;
(b) a polypeptide which consists of an amino acid sequence having an identity of at least 80% with the amino acid sequence of SEQ ID NO: 17, has a transmembrane domain, and can be localized in intracellular vesicles (for example, trans-golgi networks) or exosomes;
(c) a polypeptide which consists of an amino acid sequence obtained by deletion, substitution, addition or insertion of one or several amino acids with respect to the amino acid sequence of SEQ ID NO: 17, has a transmembrane domain, and can be localized in intracellular vesicles (for example, trans-golgi networks) or exosomes;
(d) a polypeptide which consists of an amino acid sequence encoded by a splicing variant of a polynucleotide consisting of the nucleotide sequence of SEQ ID NO: 8, has a transmembrane domain, and can be localized in intracellular vesicles (for example, trans-golgi networks) or exosomes; and
(e) a polypeptide which is a fragment of any of the polypeptides (a) to (d), has a transmembrane domain, and can be localized in intracellular vesicles (for example, trans-golgi networks) or exosomes.

Examples of the splicing variant of the STX16 gene consisting of the nucleotide sequence of SEQ ID NO: 8 include variants which are deposited as NM_001134772.3, NM_1134773.3, NM_003763.6 and NM_001204868.2 and encode proteins deposited, respectively, as NP_001128244.1, NP_001128245.1, NP_003754.2 and NP_001191797.1 in RefSeq of NCBI.

Specific examples of STX5 include the following:
(a) a polypeptide consisting of the amino acid sequence of SEQ ID NO: 18;
(b) a polypeptide which consists of an amino acid sequence having an identity of at least 80% with the amino acid sequence of SEQ ID NO: 18, has a transmembrane domain, and can be localized in intracellular vesicles (for example, golgi bodies) or exosomes;
(c) a polypeptide which consists of an amino acid sequence obtained by deletion, substitution, addition or insertion of one or several amino acids with respect to the amino acid sequence of SEQ ID NO: 18, has a transmembrane domain, and can be localized in intracellular vesicles (for example, golgi bodies) or exosomes;
(d) a polypeptide which consists of an amino acid sequence encoded by a splicing variant of a polynucleotide consisting of the nucleotide sequence of SEQ ID NO: 9, has a transmembrane domain, and can be localized in intracellular vesicles (for example, golgi bodies) or exosomes; and
(e) a polypeptide which is a fragment of any of the polypeptides (a) to (d), has a transmembrane domain, and can be localized in intracellular vesicles (for example, golgi bodies) or exosomes.

Examples of the splicing variant of the STX5 gene consisting of the nucleotide sequence of SEQ ID NO: 9 include variants which are deposited as NM_001244666.3 and NM_001330294.2 and encode proteins deposited, respectively, as NP_001231595.1 and NP_001317223.1 in RefSeq of NCBI.

Specific examples of GOSR1 include the following:
(a) a polypeptide consisting of the amino acid sequence of SEQ ID NO: 83;
(b) a polypeptide which consists of an amino acid sequence having an identity of at least 80% with the amino acid sequence of SEQ ID NO: 83, has a transmembrane domain, and can be localized in intracellular vesicles (for example, golgi bodies or trans-golgi networks) or exosomes;
(c) a polypeptide which consists of an amino acid sequence obtained by deletion, substitution, addition or insertion of one or several amino acids with respect to the amino acid sequence of SEQ ID NO: 83, has a transmembrane domain, and can be localized in intracellular vesicles (for example, golgi bodies or trans-golgi networks) or exosomes;
(d) a polypeptide which consists of an amino acid sequence encoded by a splicing variant of a polynucleotide consisting of the nucleotide sequence of SEQ ID NO: 71, has a transmembrane domain, and can be localized in intracellular vesicles (for example, golgi bodies or trans-golgi networks) or exosomes; and
(e) a polypeptide which is a fragment of any of the polypeptides (a) to (d), has a transmembrane domain, and can be localized in intracellular vesicles (for example, golgi bodies or trans-golgi networks) or exosomes.

Examples of the splicing variant of the GOSR1 gene consisting of the nucleotide sequence of SEQ ID NO: 71 include variants which are deposited as NM_001007025.2 and NM_001007024.2 and encode proteins deposited, respectively, as NP_001007026.1 and NP_001007025.1 in RefSeq of NCBI.

Specific examples of STX8 include the following:
(a) a polypeptide consisting of the amino acid sequence of SEQ ID NO: 84;
(b) a polypeptide which consists of an amino acid sequence having an identity of at least 80% with the amino acid sequence of SEQ ID NO: 84, has a transmembrane domain, and can be localized in intracellular vesicles (for example, early endosomes or late endosomes) or exosomes;
(c) a polypeptide which consists of an amino acid sequence obtained by deletion, substitution, addition or insertion of one or several amino acids with respect to the amino acid sequence of SEQ ID NO: 84, has a transmembrane domain, and can be localized in intracellular vesicles (for example, early endosomes or late endosomes) or exosomes; and
(e) a polypeptide which is a fragment of any of the polypeptides (a) to (c), has a transmembrane domain, and can be localized in intracellular vesicles (for example, early endosomes or late endosomes) or exosomes.

Specific examples of STX12 include the following:
(a) a polypeptide consisting of the amino acid sequence of SEQ ID NO: 85;
(b) a polypeptide which consists of an amino acid sequence having an identity of at least 80% with the amino acid sequence of SEQ ID NO: 85, has a transmembrane domain, and can be localized in intracellular vesicles (for example, golgi bodies, early endosomes or recycling endosomes) or exosomes;
(c) a polypeptide which consists of an amino acid sequence obtained by deletion, substitution, addition or insertion of one or several amino acids with respect to the amino acid sequence of SEQ ID NO: 85, has a transmembrane domain, and can be localized in intracellular vesicles (for example, golgi bodies, early endosomes or recycling endosomes) or exosomes; and
(e) a polypeptide which is a fragment of any of the polypeptides (a) to (c), has a transmembrane domain, and can be localized in intracellular vesicles (for example, golgi bodies, early endosomes or recycling endosomes) or exosomes.

Specific examples of VAMP8 include the following:
(a) a polypeptide consisting of the amino acid sequence of SEQ ID NO: 86;
(b) a polypeptide which consists of an amino acid sequence having an identity of at least 80% with the amino acid sequence of SEQ ID NO: 86, has a transmembrane domain, and can be localized in intracellular vesicles (for example, lysosomes, early endosomes or late endosomes) or exosomes;
(c) a polypeptide which consists of an amino acid sequence obtained by deletion, substitution, addition or insertion of one or several amino acids with respect to the amino acid sequence of SEQ ID NO: 86, has a transmembrane domain, and can be localized in intracellular vesicles (for example, lysosomes, early endosomes or late endosomes) or exosomes; and
(e) a polypeptide which is a fragment of any of the polypeptides (a) to (c), has a transmembrane domain, and can be localized in intracellular vesicles (for example, lysosomes, early endosomes or late endosomes) or exosomes.

Specific examples of SEC22B include the following:
(a) a polypeptide consisting of the amino acid sequence of SEQ ID NO: 87;
(b) a polypeptide which consists of an amino acid sequence having an identity of at least 80% with the amino acid sequence of SEQ ID NO: 87, has a transmembrane domain, and can be localized in intracellular vesicles (for example, endoplasmic reticula, endoplasmic reticulum-golgi intermediate compartments, golgi bodies, cis-golgi networks or trans-golgi networks) or exosomes;
(c) a polypeptide which consists of an amino acid sequence obtained by deletion, substitution, addition or insertion of one or several amino acids with respect to the amino acid sequence of SEQ ID NO: 87, has a transmembrane domain, and can be localized in intracellular vesicles (for example, endoplasmic reticula, endoplasmic reticulum-golgi intermediate compartments, golgi bodies, cis-golgi networks or trans-golgi networks) or exosomes; and
(e) a polypeptide which is a fragment of any of the polypeptides (a) to (c), has a transmembrane domain, and can be localized in intracellular vesicles (for example, endoplasmic reticula, endoplasmic reticulum-golgi intermediate compartments, golgi bodies, cis-golgi networks or trans-golgi networks) or exosomes.

Specific examples of STX6 include the following:
(a) a polypeptide consisting of the amino acid sequence of SEQ ID NO: 88;
(b) a polypeptide which consists of an amino acid sequence having an identity of at least 80% with the amino acid sequence of SEQ ID NO: 88, has a transmembrane domain, and can be localized in intracellular vesicles (for example, golgi bodies) or exosomes;
(c) a polypeptide which consists of an amino acid sequence obtained by deletion, substitution, addition or insertion of one or several amino acids with respect to the amino acid sequence of SEQ ID NO: 88, has a transmembrane domain, and can be localized in intracellular vesicles (for example, golgi bodies) or exosomes;
(d) a polypeptide which consists of an amino acid sequence encoded by a splicing variant of a polynucleotide consisting of the nucleotide sequence of SEQ ID NO: 76, has a transmembrane domain, and can be localized in intracellular vesicles (for example, golgi bodies) or exosomes; and
(e) a polypeptide which is a fragment of any of the polypeptides (a) to (d), has a transmembrane domain, and can be localized in intracellular vesicles (for example, golgi bodies) or exosomes.

Examples of the splicing variant of the STX6 gene consisting of the nucleotide sequence of SEQ ID NO: 76 include a variant which is deposited as NM_001286210.2 and encodes a protein deposited as NP_001273139.1 in RefSeq of NCBI.

Specific examples of VAMP3 include the following:
(a) a polypeptide consisting of the amino acid sequence of SEQ ID NO: 89;
(b) a polypeptide which consists of an amino acid sequence having an identity of at least 80% with the amino acid sequence of SEQ ID NO: 89, has a transmembrane domain, and can be localized in intracellular vesicles (for example, golgi bodies) or exosomes;
(c) a polypeptide which consists of an amino acid sequence obtained by deletion, substitution, addition or insertion of one or several amino acids with respect to the amino acid sequence of SEQ ID NO: 89, has a transmembrane domain, and can be localized in intracellular vesicles (for example, golgi bodies) or exosomes; and
(e) a polypeptide which is a fragment of one of the polypeptides (a) to (c), has a transmembrane domain, and can be localized in intracellular vesicles (for example, golgi bodies) or exosomes.

Specific examples of VAMP4 include the following:
(a) a polypeptide consisting of the amino acid sequence of SEQ ID NO: 90;
(b) a polypeptide which consists of an amino acid sequence having an identity of at least 80% with the amino acid sequence of SEQ ID NO: 90, has a transmembrane domain, and can be localized in intracellular vesicles (for example, golgi bodies or trans-golgi networks) or exosomes;
(c) a polypeptide which consists of an amino acid sequence obtained by deletion, substitution, addition or insertion of one or several amino acids with respect to the amino acid sequence of SEQ ID NO: 90, has a transmembrane domain, and can be localized in intracellular vesicles (for example, golgi bodies or trans-golgi networks) or exosomes;
(d) a polypeptide which consists of an amino acid sequence encoded by a splicing variant of a polynucleotide consisting of the nucleotide sequence of SEQ ID NO: 78, has a transmembrane domain, and can be localized in intracellular vesicles (for example, golgi bodies or trans-golgi networks) or exosomes; and
(e) a polypeptide which is a fragment of any of the polypeptides (a) to (d), has a transmembrane domain, and can be localized in intracellular vesicles (for example, golgi bodies or trans-golgi networks) or exosomes.

Examples of the splicing variant of the VAMP4 gene consisting of the nucleotide sequence of SEQ ID NO: 78 include a variant which is deposited as NM_001185127.2 and encodes a protein deposited as NP_001172056.1 in RefSeq of NCBI.

Specific examples of VTI1B include the following:
(a) a polypeptide consisting of the amino acid sequence of SEQ ID NO: 91;
(b) a polypeptide which consists of an amino acid sequence having an identity of at least 80% with the amino acid sequence of SEQ ID NO: 91, has a transmembrane domain, and can be localized in intracellular vesicles (for example, early endosomes, late endosomes, lysosomes or recycling endosomes) or exosomes;
(c) a polypeptide which consists of an amino acid sequence obtained by deletion, substitution, addition or insertion of one or several amino acids with respect to the amino acid sequence of SEQ ID NO: 91, has a transmembrane domain, and can be localized in intracellular vesicles (for example, early endosomes, late endosomes, lysosomes or recycling endosomes) or exosomes; and
(e) a polypeptide which is a fragment of any of the polypeptides (a) to (c), has a transmembrane domain, and can be localized in intracellular vesicles (for example, early endosomes, late endosomes, lysosomes or recycling endosomes) or exosomes.

Specific examples of BET1 include the following:
(a) a polypeptide consisting of the amino acid sequence of SEQ ID NO: 92;
(b) a polypeptide which consists of an amino acid sequence having an identity of at least 80% with the amino acid sequence of SEQ ID NO: 92, has a transmembrane domain, and can be localized in intracellular vesicles (for example, endoplasmic reticulum, golgi bodies or cis-golgi networks) or exosomes;
(c) a polypeptide which consists of an amino acid sequence obtained by deletion, substitution, addition or insertion of one or several amino acids with respect to the amino acid sequence of SEQ ID NO: 92, has a transmembrane domain, and can be localized in intracellular vesicles (for example, endoplasmic reticulum, golgi bodies or cis-golgi networks) or exosomes;
(d) a polypeptide which consists of an amino acid sequence encoded by a splicing variant of a polynucleotide consisting of the nucleotide sequence of SEQ ID NO: 80, has a transmembrane domain, and can be localized in intracellular vesicles (for example, endoplasmic reticulum, golgi bodies or cis-golgi networks) or exosomes; and
(e) a polypeptide which is a fragment of any of the polypeptides (a) to (d), has a transmembrane domain, and can be localized in intracellular vesicles (for example, endoplasmic reticulum, golgi bodies or cis-golgi networks) or exosomes.

Examples of the splicing variant of the BET1 gene consisting of the nucleotide sequence of SEQ ID NO: 80 include a variant which is deposited as NM_001317739.2 and encodes a protein deposited as NP_001304668.1 in RefSeq of NCBI.

Specific examples of BET1L include the following:
(a) a polypeptide consisting of the amino acid sequence of SEQ ID NO: 93;
(b) a polypeptide which consists of an amino acid sequence having an identity of at least 80% with the amino acid sequence of SEQ ID NO: 93, has a transmembrane domain, and can be localized in intracellular vesicles (for example, golgi bodies or trans-golgi networks) or exosomes;
(c) a polypeptide which consists of an amino acid sequence obtained by deletion, substitution, addition or insertion of one or several amino acids with respect to the amino acid sequence of SEQ ID NO: 93, has a transmembrane domain, and can be localized in intracellular vesicles (for example, golgi bodies or trans-golgi networks) or exosomes;
(d) a polypeptide which consists of an amino acid sequence encoded by a splicing variant of a polynucleotide consisting of the nucleotide sequence of SEQ ID NO: 81, has a transmembrane domain, and can be localized in intracellular vesicles (for example, golgi bodies or trans-golgi networks) or exosomes; and
(e) a polypeptide which is a fragment of any of the polypeptides (a) to (d), has a transmembrane domain, and can be localized in intracellular vesicles (for example, golgi bodies or trans-golgi networks) or exosomes.

Examples of the splicing variant of the BET1L gene consisting of the nucleotide sequence of SEQ ID NO: 81 include variants which are deposited as NM_001098787.2 and NM_016526.5 and encode proteins deposited, respectively, as NP_001092257.1 and NP_057610.2 in RefSeq of NCBI.

Specific examples of USE1 include the following:
(a) a polypeptide consisting of the amino acid sequence of SEQ ID NO: 94;
(b) a polypeptide which consists of an amino acid sequence having an identity of at least 80% with the amino acid sequence of SEQ ID NO: 94, has a transmembrane domain, and can be localized in intracellular vesicles (for example, endoplasmic reticula) or exosomes;
(c) a polypeptide which consists of an amino acid sequence obtained by deletion, substitution, addition or insertion of one or several amino acids with respect to the amino acid sequence of SEQ ID NO: 94, has a transmembrane domain, and can be localized in intracellular vesicles (for example, endoplasmic reticula) or exosomes; and
(e) a polypeptide which is a fragment of any of the polypeptides (a) to (c), has a transmembrane domain, and can be localized in intracellular vesicles (for example, endoplasmic reticula) or exosomes.

The polynucleotide encoding a SNARE protein for use in the present invention includes polynucleotides encoding the SNARE protein, and polynucleotides encoding a polypeptide functionally equivalent to the SNARE protein. Specific examples of the polynucleotide encoding a polypeptide functionally equivalent to the SNARE protein include the following polynucleotides (g) to (k) and (m) to (o) or (g), (h), (j), (k) and (m) to (o) .

Specific examples of the polynucleotide encoding VAMP7 include the following:
(f) a polynucleotide consisting of the nucleotide sequence of SEQ ID NO: 1;
(g) a polynucleotide which consists of a nucleotide sequence having an identity of at least 80% with the nucleotide sequence of SEQ ID NO: 1, and encodes a polypeptide which has a transmembrane domain, and can be localized in intracellular vesicles (for example, late endosomes or lysosomes) or exosomes;
(h) a polynucleotide which consists of a nucleotide sequence obtained by deletion, substitution, addition or insertion of one or several nucleotides with respect to the nucleotide sequence of SEQ ID NO: 1, and encodes a polypeptide which has a transmembrane domain, and can be localized in intracellular vesicles (for example, late endosomes or lysosomes) or exosomes;
(i) a polynucleotide which is a splicing variant of a polynucleotide consisting of the nucleotide sequence of SEQ ID NO: 1, and encodes a polypeptide which has a transmembrane domain, and can be localized in intracellular vesicles (for example, late endosomes or lysosomes) or exosomes;
(j) a polynucleotide which is hybridized under stringent conditions to a complementary strand of a polynucleotide consisting of the nucleotide sequence of SEQ ID NO: 1, and encodes a polypeptide which has a transmembrane domain, and can be localized in intracellular vesicles (for example, late endosomes or lysosomes) or exosomes;
(k) a polynucleotide which is a fragment of any of the polynucleotides (f) to (j), and encodes a polypeptide which has a transmembrane domain, and can be localized in intracellular vesicles (for example, late endosomes or lysosomes) or exosomes;
(l) a polynucleotide encoding a polypeptide consisting of the amino acid sequence of SEQ ID NO: 10;
(m) a polynucleotide encoding a polypeptide which consists of an amino acid sequence having an identity of at least 80% with the amino acid sequence of SEQ ID NO: 10, has a transmembrane domain, and can be localized in intracellular vesicles (for example, late endosomes or lysosomes) or exosomes;
(n) a polynucleotide encoding a polypeptide which consists of an amino acid sequence obtained by deletion, substitution, addition or insertion of one or several amino acids with respect to the amino acid sequence of SEQ ID NO: 10, has a transmembrane domain, and can be localized in intracellular vesicles (for example, late endosomes or lysosomes) or exosomes; and
(o) a polynucleotide encoding a polypeptide which is a fragment of any of the polypeptides in (l) to (n), has a transmembrane domain, and can be localized in intracellular vesicles (for example, late endosomes or lysosomes) or exosomes.

Specific examples of the polynucleotide encoding GOSR2 include the following:
(f) a polynucleotide consisting of the nucleotide sequence of SEQ ID NO: 2;
(g) a polynucleotide which consists of a nucleotide sequence having an identity of at least 80% with the nucleotide sequence of SEQ ID NO: 2, and encodes a polypeptide which has a transmembrane domain, and can be localized in intracellular vesicles (for example, endoplasmic reticulum-golgi intermediate compartments or golgi bodies) or exosomes;
(h) a polynucleotide which consists of a nucleotide sequence obtained by deletion, substitution, addition or insertion of one or several nucleotides with respect to the nucleotide sequence of SEQ ID NO: 2, and encodes a polypeptide which has a transmembrane domain, and can be localized in intracellular vesicles (for example, endoplasmic reticulum-golgi intermediate compartments or golgi bodies) or exosomes;
(i) a polynucleotide which is a splicing variant of a polynucleotide consisting of the nucleotide sequence of SEQ ID NO: 2, and encodes a polypeptide which has a transmembrane domain, and can be localized in intracellular vesicles (for example, endoplasmic reticulum-golgi intermediate compartments or golgi bodies) or exosomes;
(j) a polynucleotide which is hybridized under stringent conditions to a complementary strand of a polynucleotide consisting of the nucleotide sequence of SEQ ID NO: 2, and encodes a polypeptide which has a transmembrane domain, and can be localized in intracellular vesicles (for example, endoplasmic reticulum-golgi intermediate compartments or golgi bodies) or exosomes;
(k) a polynucleotide which is a fragment of any of the polynucleotides (f) to (j), and encodes a polypeptide which has a transmembrane domain, and can be localized in intracellular vesicles (for example, endoplasmic reticulum-golgi intermediate compartments or golgi bodies) or exosomes;
(l) a polynucleotide encoding a polypeptide consisting of the amino acid sequence of SEQ ID NO: 11;
(m) a polynucleotide encoding a polypeptide which consists of an amino acid sequence having an identity of at least 80% with the amino acid sequence of SEQ ID NO: 11, has a transmembrane domain, and can be localized in intracellular vesicles (for example, endoplasmic reticulum-golgi intermediate compartments or golgi bodies) or exosomes;
(n) a polynucleotide encoding a polypeptide which consists of an amino acid sequence obtained by deletion, substitution, addition or insertion of one or several amino acids with respect to the amino acid sequence of SEQ ID NO: 11, has a transmembrane domain, and can be localized in intracellular vesicles (for example, endoplasmic reticulum-golgi intermediate compartments or golgi bodies) or exosomes; and
(o) a polynucleotide encoding a polypeptide which is a fragment of any of the polypeptides in (l) to (n), has a transmembrane domain, and can be localized in intracellular vesicles (for example, endoplasmic reticulum-golgi intermediate compartments or golgi bodies) or exosomes.

Specific examples of the polynucleotide encoding STX10 include the following:
(f) a polynucleotide consisting of the nucleotide sequence of SEQ ID NO: 3;
(g) a polynucleotide which consists of a nucleotide sequence having an identity of at least 80% with the nucleotide sequence of SEQ ID NO: 3, and encodes a polypeptide which has a transmembrane domain, and can be localized in intracellular vesicles (for example, trans-golgi networks) or exosomes;
(h) a polynucleotide which consists of a nucleotide sequence obtained by deletion, substitution, addition or insertion of one or several nucleotides with respect to the nucleotide sequence of SEQ ID NO: 3, and encodes a polypeptide which has a transmembrane domain, and can be localized in intracellular vesicles (for example, trans-golgi networks) or exosomes;
(i) a polynucleotide which is a splicing variant of a polynucleotide consisting of the nucleotide sequence of SEQ ID NO: 3, and encodes a polypeptide which has a transmembrane domain, and can be localized in intracellular vesicles (for example, trans-golgi networks) or exosomes;
(j) a polynucleotide which is hybridized under stringent conditions to a complementary strand of a polynucleotide consisting of the nucleotide sequence of SEQ ID NO: 3, and encodes a polypeptide which has a transmembrane domain, and can be localized in intracellular vesicles (for example, trans-golgi networks) or exosomes;
(k) a polynucleotide which is a fragment of any of the polynucleotides (f) to (j), and encodes a polypeptide which has a transmembrane domain, and can be localized in intracellular vesicles (for example, trans-golgi networks) or exosomes;
(l) a polynucleotide encoding a polypeptide consisting of the amino acid sequence of SEQ ID NO: 12;
(m) a polynucleotide encoding a polypeptide which consists of an amino acid sequence having an identity of at least 80% with the amino acid sequence of SEQ ID NO: 12, has a transmembrane domain, and can be localized in intracellular vesicles (for example, trans-golgi networks) or exosomes;
(n) a polynucleotide encoding a polypeptide which consists of an amino acid sequence obtained by deletion, substitution, addition or insertion of one or several amino acids with respect to the amino acid sequence of SEQ ID NO: 12, has a transmembrane domain, and can be localized in intracellular vesicles (for example, trans-golgi networks) or exosomes; and
(o) a polynucleotide encoding a polypeptide which is a fragment of any of the polypeptides in (l) to (n), has a transmembrane domain, and can be localized in intracellular vesicles (for example, trans-golgi networks) or exosomes.

Specific examples of the polynucleotide encoding STX18 include the following:
(f) a polynucleotide consisting of the nucleotide sequence of SEQ ID NO: 4;
(g) a polynucleotide which consists of a nucleotide sequence having an identity of at least 80% with the nucleotide sequence of SEQ ID NO: 4, and encodes a polypeptide which has a transmembrane domain, and can be localized in intracellular vesicles (for example, endoplasmic reticula) or exosomes;
(h) a polynucleotide which consists of a nucleotide sequence obtained by deletion, substitution, addition or insertion of one or several nucleotides with respect to the nucleotide sequence of SEQ ID NO: 4, and encodes a polypeptide which has a transmembrane domain, and can be localized in intracellular vesicles (for example, endoplasmic reticula) or exosomes;
(i) a polynucleotide which is a splicing variant of a polynucleotide consisting of the nucleotide sequence of SEQ ID NO: 4, and encodes a polypeptide which has a transmembrane domain, and can be localized in intracellular vesicles (for example, endoplasmic reticula) or exosomes;
(j) a polynucleotide which is hybridized under stringent conditions to a complementary strand of a polynucleotide consisting of the nucleotide sequence of SEQ ID NO: 4, and encodes a polypeptide which has a transmembrane domain, and can be localized in intracellular vesicles (for example, endoplasmic reticula) or exosomes;
(k) a polynucleotide which is a fragment of any of the polynucleotides (f) to (j), and encodes a polypeptide which has a transmembrane domain, and can be localized in intracellular vesicles (for example, endoplasmic reticula) or exosomes;
(l) a polynucleotide encoding a polypeptide consisting of the amino acid sequence of SEQ ID NO: 13;
(m) a polynucleotide encoding a polypeptide which consists of an amino acid sequence having an identity of at least 80% with the amino acid sequence of SEQ ID NO: 13, has a transmembrane domain, and can be localized in intracellular vesicles (for example, endoplasmic reticula) or exosomes;
(n) a polynucleotide encoding a polypeptide which consists of an amino acid sequence obtained by deletion, substitution, addition or insertion of one or several amino acids with respect to the amino acid sequence of SEQ ID NO: 13, has a transmembrane domain, and can be localized in intracellular vesicles (for example, endoplasmic reticula) or exosomes; and
(o) a polynucleotide encoding a polypeptide which is a fragment of any of the polypeptides in (l) to (n), has a transmembrane domain, and can be localized in intracellular vesicles (for example, endoplasmic reticula) or exosomes.

Specific examples of the polynucleotide encoding BNIP1 include the following:
(f) a polynucleotide consisting of the nucleotide sequence of SEQ ID NO: 5;
(g) a polynucleotide which consists of a nucleotide sequence having an identity of at least 80% with the nucleotide sequence of SEQ ID NO: 5, and encodes a polypeptide which has a transmembrane domain, and can be localized in intracellular vesicles (for example, endoplasmic reticula) or exosomes;
(h) a polynucleotide which consists of a nucleotide sequence obtained by deletion, substitution, addition or insertion of one or several nucleotides with respect to the nucleotide sequence of SEQ ID NO: 5, and encodes a polypeptide which has a transmembrane domain, and can be localized in intracellular vesicles (for example, endoplasmic reticula) or exosomes;
(i) a polynucleotide which is a splicing variant of a polynucleotide consisting of the nucleotide sequence of SEQ ID NO: 5, and encodes a polypeptide which has a transmembrane domain, and can be localized in intracellular vesicles (for example, endoplasmic reticula) or exosomes;
(j) a polynucleotide which is hybridized under stringent conditions to a complementary strand of a polynucleotide consisting of the nucleotide sequence of SEQ ID NO: 5, and encodes a polypeptide which has a transmembrane domain, and can be localized in intracellular vesicles (for example, endoplasmic reticula) or exosomes;
(k) a polynucleotide which is a fragment of any of the polynucleotides (f) to (j), and encodes a polypeptide which has a transmembrane domain, and can be localized in intracellular vesicles (for example, endoplasmic reticula) or exosomes;
(l) a polynucleotide encoding a polypeptide consisting of the amino acid sequence of SEQ ID NO: 14;
(m) a polynucleotide encoding a polypeptide which consists of an amino acid sequence having an identity of at least 80% with the amino acid sequence of SEQ ID NO: 14, has a transmembrane domain, and can be localized in intracellular vesicles (for example, endoplasmic reticula) or exosomes;
(n) a polynucleotide encoding a polypeptide which consists of an amino acid sequence obtained by deletion, substitution, addition or insertion of one or several amino acids with respect to the amino acid sequence of SEQ ID NO: 14, has a transmembrane domain, and can be localized in intracellular vesicles (for example, endoplasmic reticula) or exosomes; and
(o) a polynucleotide encoding a polypeptide which is a fragment of any of the polypeptides in (l) to (n), has a transmembrane domain, and can be localized in intracellular vesicles (for example, endoplasmic reticula) or exosomes.

Specific examples of the polynucleotide encoding STX7 include the following:
(f) a polynucleotide consisting of the nucleotide sequence of SEQ ID NO: 6;
(g) a polynucleotide which consists of a nucleotide sequence having an identity of at least 80% with the nucleotide sequence of SEQ ID NO: 6, and encodes a polypeptide which has a transmembrane domain, and can be localized in intracellular vesicles (for example, early endosomes or late endosomes) or exosomes;
(h) a polynucleotide which consists of a nucleotide sequence obtained by deletion, substitution, addition or insertion of one or several nucleotides with respect to the nucleotide sequence of SEQ ID NO: 6, and encodes a polypeptide which has a transmembrane domain, and can be localized in intracellular vesicles (for example, early endosomes or late endosomes) or exosomes;
(i) a polynucleotide which is a splicing variant of a polynucleotide consisting of the nucleotide sequence of SEQ ID NO: 6, and encodes a polypeptide which has a transmembrane domain, and can be localized in intracellular vesicles (for example, early endosomes or late endosomes) or exosomes;
(j) a polynucleotide which is hybridized under stringent conditions to a complementary strand of a polynucleotide consisting of the nucleotide sequence of SEQ ID NO: 6, and encodes a polypeptide which has a transmembrane domain, and can be localized in intracellular vesicles (for example, early endosomes or late endosomes) or exosomes;
(k) a polynucleotide which is a fragment of any of the polynucleotides (f) to (j), and encodes a polypeptide which has a transmembrane domain, and can be localized in intracellular vesicles (for example, early endosomes or late endosomes) or exosomes;
(l) a polynucleotide encoding a polypeptide consisting of the amino acid sequence of SEQ ID NO: 15;
(m) a polynucleotide encoding a polypeptide which consists of an amino acid sequence having an identity of at least 80% with the amino acid sequence of SEQ ID NO: 15, has a transmembrane domain, and can be localized in intracellular vesicles (for example, early endosomes or late endosomes) or exosomes;
(n) a polynucleotide encoding a polypeptide which consists of an amino acid sequence obtained by deletion, substitution, addition or insertion of one or several amino acids with respect to the amino acid sequence of SEQ ID NO: 15, has a transmembrane domain, and can be localized in intracellular vesicles (for example, early endosomes or late endosomes) or exosomes; and
(o) a polynucleotide encoding a polypeptide which is a fragment of any of the polypeptides in (l) to (n), has a transmembrane domain, and can be localized in intracellular vesicles (for example, early endosomes or late endosomes) or exosomes.

Specific examples of the polynucleotide encoding VTI1A include the following:
(f) a polynucleotide consisting of the nucleotide sequence of SEQ ID NO: 7;
(g) a polynucleotide which consists of a nucleotide sequence having an identity of at least 80% with the nucleotide sequence of SEQ ID NO: 7, and encodes a polypeptide which has a transmembrane domain, and can be localized in intracellular vesicles (for example, trans-golgi networks) or exosomes;
(h) a polynucleotide which consists of a nucleotide sequence obtained by deletion, substitution, addition or insertion of one or several nucleotides with respect to the nucleotide sequence of SEQ ID NO: 7, and encodes a polypeptide which has a transmembrane domain, and can be localized in intracellular vesicles (for example, trans-golgi networks) or exosomes;
(i) a polynucleotide which is a splicing variant of a polynucleotide consisting of the nucleotide sequence of SEQ ID NO: 7, and encodes a polypeptide which has a transmembrane domain, and can be localized in intracellular vesicles (for example, trans-golgi networks) or exosomes;
(j) a polynucleotide which is hybridized under stringent conditions to a complementary strand of a polynucleotide consisting of the nucleotide sequence of SEQ ID NO: 7, and encodes a polypeptide which has a transmembrane domain, and can be localized in intracellular vesicles (for example, trans-golgi networks) or exosomes;
(k) a polynucleotide which is a fragment of any of the polynucleotides (f) to (j), and encodes a polypeptide which has a transmembrane domain, and can be localized in intracellular vesicles (for example, trans-golgi networks) or exosomes;
(l) a polynucleotide encoding a polypeptide consisting of the amino acid sequence of SEQ ID NO: 16;
(m) a polynucleotide encoding a polypeptide which consists of an amino acid sequence having an identity of at least 80% with the amino acid sequence of SEQ ID NO: 16, has a transmembrane domain, and can be localized in intracellular vesicles (for example, trans-golgi networks) or exosomes;
(n) a polynucleotide encoding a polypeptide which consists of an amino acid sequence obtained by deletion, substitution, addition or insertion of one or several amino acids with respect to the amino acid sequence of SEQ ID NO: 16, has a transmembrane domain, and can be localized in intracellular vesicles (for example, trans-golgi networks) or exosomes; and
(o) a polynucleotide encoding a polypeptide which is a fragment of any of the polypeptides in (l) to (n), has a transmembrane domain, and can be localized in intracellular vesicles (for example, trans-golgi networks) or exosomes.

Specific examples of the polynucleotide encoding STX16 include the following:
(f) a polynucleotide consisting of the nucleotide sequence of SEQ ID NO: 8;
(g) a polynucleotide which consists of a nucleotide sequence having an identity of at least 80% with the nucleotide sequence of SEQ ID NO: 8, and encodes a polypeptide which has a transmembrane domain, and can be localized in intracellular vesicles (for example, trans-golgi networks) or exosomes;
(h) a polynucleotide which consists of a nucleotide sequence obtained by deletion, substitution, addition or insertion of one or several nucleotides with respect to the nucleotide sequence of SEQ ID NO: 8, and encodes a polypeptide which has a transmembrane domain, and can be localized in intracellular vesicles (for example, trans-golgi networks) or exosomes;
(i) a polynucleotide which is a splicing variant of a polynucleotide consisting of the nucleotide sequence of SEQ ID NO: 8, and encodes a polypeptide which has a transmembrane domain, and can be localized in intracellular vesicles (for example, trans-golgi networks) or exosomes;
(j) a polynucleotide which is hybridized under stringent conditions to a complementary strand of a polynucleotide consisting of the nucleotide sequence of SEQ ID NO: 8, and encodes a polypeptide which has a transmembrane domain, and can be localized in intracellular vesicles (for example, trans-golgi networks) or exosomes;
(k) a polynucleotide which is a fragment of any of the polynucleotides (f) to (j), and encodes a polypeptide which has a transmembrane domain, and can be localized in intracellular vesicles (for example, trans-golgi networks) or exosomes;
(l) a polynucleotide encoding a polypeptide consisting of the amino acid sequence of SEQ ID NO: 17;
(m) a polynucleotide encoding a polypeptide which consists of an amino acid sequence having an identity of at least 80% with the amino acid sequence of SEQ ID NO: 17, has a transmembrane domain, and can be localized in intracellular vesicles (for example, trans-golgi networks) or exosomes;
(n) a polynucleotide encoding a polypeptide which consists of an amino acid sequence obtained by deletion, substitution, addition or insertion of one or several amino acids with respect to the amino acid sequence of SEQ ID NO: 17, has a transmembrane domain, and can be localized in intracellular vesicles (for example, trans-golgi networks) or exosomes; and
(o) a polynucleotide encoding a polypeptide which is a fragment of any of the polypeptides in (l) to (n), has a transmembrane domain, and can be localized in intracellular vesicles (for example, trans-golgi networks) or exosomes.

Specific examples of the polynucleotide encoding STX5 include the following:
(f) a polynucleotide consisting of the nucleotide sequence of SEQ ID NO: 9;
(g) a polynucleotide which consists of a nucleotide sequence having an identity of at least 80% with the nucleotide sequence of SEQ ID NO: 9, and encodes a polypeptide which has a transmembrane domain, and can be localized in intracellular vesicles (for example, golgi bodies) or exosomes;
(h) a polynucleotide which consists of a nucleotide sequence obtained by deletion, substitution, addition or insertion of one or several nucleotides with respect to the nucleotide sequence of SEQ ID NO: 9, and encodes a polypeptide which has a transmembrane domain, and can be localized in intracellular vesicles (for example, golgi bodies) or exosomes;
(i) a polynucleotide which is a splicing variant of a polynucleotide consisting of the nucleotide sequence of SEQ ID NO: 9, and encodes a polypeptide which has a transmembrane domain, and can be localized in intracellular vesicles (for example, golgi bodies) or exosomes;
(j) a polynucleotide which is hybridized under stringent conditions to a complementary strand of a polynucleotide consisting of the nucleotide sequence of SEQ ID NO: 9, and encodes a polypeptide which has a transmembrane domain, and can be localized in intracellular vesicles (for example, golgi bodies) or exosomes;
(k) a polynucleotide which is a fragment of any of the polynucleotides (f) to (j), and encodes a polypeptide which has a transmembrane domain, and can be localized in intracellular vesicles (for example, golgi bodies) or exosomes;
(l) a polynucleotide encoding a polypeptide consisting of the amino acid sequence of SEQ ID NO: 18;
(m) a polynucleotide encoding a polypeptide which consists of an amino acid sequence having an identity of at least 80% with the amino acid sequence of SEQ ID NO: 18, has a transmembrane domain, and can be localized in intracellular vesicles (for example, golgi bodies) or exosomes;
(n) a polynucleotide encoding a polypeptide which consists of an amino acid sequence obtained by deletion, substitution, addition or insertion of one or several amino acids with respect to the amino acid sequence of SEQ ID NO: 18, has a transmembrane domain, and can be localized in intracellular vesicles (for example, golgi bodies) or exosomes; and
(o) a polynucleotide encoding a polypeptide which is a fragment of any of the polypeptides in (l) to (n), has a transmembrane domain, and can be localized in intracellular vesicles (for example, golgi bodies) or exosomes.

Specific examples of the polynucleotide encoding GOSR1 include the following:
(f) a polynucleotide consisting of the nucleotide sequence of SEQ ID NO: 71;
(g) a polynucleotide which consists of a nucleotide sequence having an identity of at least 80% with the nucleotide sequence of SEQ ID NO: 71, and encodes a polypeptide which has a transmembrane domain, and can be localized in intracellular vesicles (for example, golgi bodies or trans-golgi networks) or exosomes;
(h) a polynucleotide which consists of a nucleotide sequence obtained by deletion, substitution, addition or insertion of one or several nucleotides with respect to the nucleotide sequence of SEQ ID NO: 71, and encodes a polypeptide which has a transmembrane domain, and can be localized in intracellular vesicles (for example, golgi bodies or trans-golgi networks) or exosomes;
(i) a polynucleotide which is a splicing variant of a polynucleotide consisting of the nucleotide sequence of SEQ ID NO: 71, and encodes a polypeptide which has a transmembrane domain, and can be localized in intracellular vesicles (for example, golgi bodies or trans-golgi networks) or exosomes;
(j) a polynucleotide which is hybridized under stringent conditions to a complementary strand of a polynucleotide consisting of the nucleotide sequence of SEQ ID NO: 71, and encodes a polypeptide which has a transmembrane domain, and can be localized in intracellular vesicles (for example, golgi bodies or trans-golgi networks) or exosomes;
(k) a polynucleotide which is a fragment of any of the polynucleotides (f) to (j), and encodes a polypeptide which has a transmembrane domain, and can be localized in intracellular vesicles (for example, golgi bodies or trans-golgi networks) or exosomes;
(l) a polynucleotide encoding a polypeptide consisting of the amino acid sequence of SEQ ID NO: 83;
(m) a polynucleotide encoding a polypeptide which consists of an amino acid sequence having an identity of at least 80% with the amino acid sequence of SEQ ID NO: 83, has a transmembrane domain, and can be localized in intracellular vesicles (for example, golgi bodies or trans-golgi networks) or exosomes;
(n) a polynucleotide encoding a polypeptide which consists of an amino acid sequence obtained by deletion, substitution, addition or insertion of one or several amino acids with respect to the amino acid sequence of SEQ ID NO: 83, has a transmembrane domain, and can be localized in intracellular vesicles (for example, golgi bodies or trans-golgi networks) or exosomes; and (o) a polynucleotide encoding a polypeptide which is a fragment of any of the polypeptides in (l) to (n), has a transmembrane domain, and can be localized in intracellular vesicles (for example, golgi bodies or trans-golgi networks) or exosomes.

Specific examples of the polynucleotide encoding STX8 include the following:
(f) a polynucleotide consisting of the nucleotide sequence of SEQ ID NO: 72;
(g) a polynucleotide which consists of a nucleotide sequence having an identity of at least 80% with the nucleotide sequence of SEQ ID NO: 72, and encodes a polypeptide which has a transmembrane domain, and can be localized in intracellular vesicles (for example, early endosomes or late endosomes) or exosomes;
(h) a polynucleotide which consists of a nucleotide sequence obtained by deletion, substitution, addition or insertion of one or several nucleotides with respect to the nucleotide sequence of SEQ ID NO: 72, and encodes a polypeptide which has a transmembrane domain, and can be localized in intracellular vesicles (for example, early endosomes or late endosomes) or exosomes;
(j) a polynucleotide which is hybridized under stringent conditions to a complementary strand of a polynucleotide consisting of the nucleotide sequence of SEQ ID NO: 72, and encodes a polypeptide which has a transmembrane domain, and can be localized in intracellular vesicles (for example, early endosomes or late endosomes) or exosomes;
(k) a polynucleotide which is a fragment of any of the polynucleotides (f) to (h) and (j), and encodes a polypeptide which has a transmembrane domain, and can be localized in intracellular vesicles (for example, early endosomes or late endosomes) or exosomes;
(l) a polynucleotide encoding a polypeptide consisting of the amino acid sequence of SEQ ID NO: 84;
(m) a polynucleotide encoding a polypeptide which consists of an amino acid sequence having an identity of at least 80% with the amino acid sequence of SEQ ID NO: 84, has a transmembrane domain, and can be localized in intracellular vesicles (for example, early endosomes or late endosomes) or exosomes;
(n) a polynucleotide encoding a polypeptide which consists of an amino acid sequence obtained by deletion, substitution, addition or insertion of one or several amino acids with respect to the amino acid sequence of SEQ ID NO: 84, has a transmembrane domain, and can be localized in intracellular vesicles (for example, early endosomes or late endosomes) or exosomes; and (o) a polynucleotide encoding a polypeptide which is a fragment of any of the polypeptides in (l) to (n), has a transmembrane domain, and can be localized in intracellular vesicles (for example, early endosomes or late endosomes) or exosomes.

Specific examples of the polynucleotide encoding STX12 include the following:
(f) a polynucleotide consisting of the nucleotide sequence of SEQ ID NO: 73;
(g) a polynucleotide which consists of a nucleotide sequence having an identity of at least 80% with the nucleotide sequence of SEQ ID NO: 73, and encodes a polypeptide which has a transmembrane domain, and can be localized in intracellular vesicles (for example, golgi bodies, early endosomes or recycling endosomes) or exosomes;
(h) a polynucleotide which consists of a nucleotide sequence obtained by deletion, substitution, addition or insertion of one or several nucleotides with respect to the nucleotide sequence of SEQ ID NO: 73, and encodes a polypeptide which has a transmembrane domain, and can be localized in intracellular vesicles (for example, golgi bodies, early endosomes or recycling endosomes) or exosomes;
(j) a polynucleotide which is hybridized under stringent conditions to a complementary strand of a polynucleotide consisting of the nucleotide sequence of SEQ ID NO: 73, and encodes a polypeptide which has a transmembrane domain, and can be localized in intracellular vesicles (for example, golgi bodies, early endosomes or recycling endosomes) or exosomes;
(k) a polynucleotide which is a fragment of any of the polynucleotides (f) to (h) and (j), and encodes a polypeptide which has a transmembrane domain, and can be localized in intracellular vesicles (for example, golgi bodies, early endosomes or recycling endosomes) or exosomes;
(l) a polynucleotide encoding a polypeptide consisting of the amino acid sequence of SEQ ID NO: 85;
(m) a polynucleotide encoding a polypeptide which consists of an amino acid sequence having an identity of at least 80% with the amino acid sequence of SEQ ID NO: 85, has a transmembrane domain, and can be localized in intracellular vesicles (for example, golgi bodies, early endosomes or recycling endosomes) or exosomes;
(n) a polynucleotide encoding a polypeptide which consists of an amino acid sequence obtained by deletion, substitution, addition or insertion of one or several amino acids with respect to the amino acid sequence of SEQ ID NO: 85, has a transmembrane domain, and can be localized in intracellular vesicles (for example, golgi bodies, early endosomes or recycling endosomes) or exosomes; and
(o) a polynucleotide encoding a polypeptide which is a fragment of any of the polypeptides in (l) to (n), has a transmembrane domain, and can be localized in intracellular vesicles (for example, golgi bodies, early endosomes or recycling endosomes) or exosomes.

Specific examples of the polynucleotide encoding VAMP8 include the following:
(f) a polynucleotide consisting of the nucleotide sequence of SEQ ID NO: 74;
(g) a polynucleotide which consists of a nucleotide sequence having an identity of at least 80% with the nucleotide sequence of SEQ ID NO: 74, and encodes a polypeptide which has a transmembrane domain, and can be localized in intracellular vesicles (for example, lysosomes, early endosomes or late endosomes) or exosomes;
(h) a polynucleotide which consists of a nucleotide sequence obtained by deletion, substitution, addition or insertion of one or several nucleotides with respect to the nucleotide sequence of SEQ ID NO: 74, and encodes a polypeptide which has a transmembrane domain, and can be localized in intracellular vesicles (for example, lysosomes, early endosomes or late endosomes) or exosomes;
(j) a polynucleotide which is hybridized under stringent conditions to a complementary strand of a polynucleotide consisting of the nucleotide sequence of SEQ ID NO: 74, and encodes a polypeptide which has a transmembrane domain, and can be localized in intracellular vesicles (for example, lysosomes, early endosomes or late endosomes) or exosomes;
(k) a polynucleotide which is a fragment of any of the polynucleotides (f) to (h) and (j), and encodes a polypeptide which has a transmembrane domain, and can be localized in intracellular vesicles (for example, lysosomes, early endosomes or late endosomes) or exosomes;
(l) a polynucleotide encoding a polypeptide consisting of the amino acid sequence of SEQ ID NO: 86;
(m) a polynucleotide encoding a polypeptide which consists of an amino acid sequence having an identity of at least 80% with the amino acid sequence of SEQ ID NO: 86, has a transmembrane domain, and can be localized in intracellular vesicles (for example, lysosomes, early endosomes or late endosomes) or exosomes;
(n) a polynucleotide encoding a polypeptide which consists of an amino acid sequence obtained by deletion, substitution, addition or insertion of one or several amino acids with respect to the amino acid sequence of SEQ ID NO: 86, has a transmembrane domain, and can be localized in intracellular vesicles (for example, lysosomes, early endosomes or late endosomes) or exosomes; and
(o) a polynucleotide encoding a polypeptide which is a fragment of any of the polypeptides in (l) to (n), has a transmembrane domain, and can be localized in intracellular vesicles (for example, lysosomes, early endosomes or late endosomes) or exosomes.

Specific examples of the polynucleotide encoding SEC22B include the following:
(f) a polynucleotide consisting of the nucleotide sequence of SEQ ID NO: 75;
(g) a polynucleotide which consists of a nucleotide sequence having an identity of at least 80% with the nucleotide sequence of SEQ ID NO: 75, and encodes a polypeptide which has a transmembrane domain, and can be localized in intracellular vesicles (for example, endoplasmic reticula, endoplasmic reticulum-golgi intermediate compartments, golgi bodies, cis-golgi networks or trans-golgi networks) or exosomes;
(h) a polynucleotide which consists of a nucleotide sequence obtained by deletion, substitution, addition or insertion of one or several nucleotides with respect to the nucleotide sequence of SEQ ID NO: 75, and encodes a polypeptide which has a transmembrane domain, and can be localized in intracellular vesicles (for example, endoplasmic reticula, endoplasmic reticulum-golgi intermediate compartments, golgi bodies, cis-golgi networks or trans-golgi networks) or exosomes;
(j) a polynucleotide which is hybridized under stringent conditions to a complementary strand of a polynucleotide consisting of the nucleotide sequence of SEQ ID NO: 75, and encodes a polypeptide which has a transmembrane domain, and can be localized in intracellular vesicles (for example, endoplasmic reticula, endoplasmic reticulum-golgi intermediate compartments, golgi bodies, cis-golgi networks or trans-golgi networks) or exosomes; (k) a polynucleotide which is a fragment of any of the polynucleotides (f) to (h) and (j), and encodes a polypeptide which has a transmembrane domain, and can be localized in intracellular vesicles (for example, endoplasmic reticula, endoplasmic reticulum-golgi intermediate compartments, golgi bodies, cis-golgi networks or trans-golgi networks) or exosomes;
(l) a polynucleotide encoding a polypeptide consisting of the amino acid sequence of SEQ ID NO: 87;
(m) a polynucleotide encoding a polypeptide which consists of an amino acid sequence having an identity of at least 80% with the amino acid sequence of SEQ ID NO: 87, has a transmembrane domain, and can be localized in intracellular vesicles (for example, endoplasmic reticula, endoplasmic reticulum-golgi intermediate compartments, golgi bodies, cis-golgi networks or trans-golgi networks) or exosomes;
(n) a polynucleotide encoding a polypeptide which consists of an amino acid sequence obtained by deletion, substitution, addition or insertion of one or several amino acids with respect to the amino acid sequence of SEQ ID NO: 87, has a transmembrane domain, and can be localized in intracellular vesicles (for example, endoplasmic reticula, endoplasmic reticulum-golgi intermediate compartments, golgi bodies, cis-golgi networks or trans-golgi networks) or exosomes; and (o) a polynucleotide encoding a polypeptide which is a fragment of any of the polypeptides in (l) to (n), has a transmembrane domain, and can be localized in intracellular vesicles (for example, endoplasmic reticula, endoplasmic reticulum-golgi intermediate compartments, golgi bodies, cis-golgi networks or trans-golgi networks) or exosomes.

Specific examples of the polynucleotide encoding STX6 include the following:
(f) a polynucleotide consisting of the nucleotide sequence of SEQ ID NO: 76;
(g) a polynucleotide which consists of a nucleotide sequence having an identity of at least 80% with the nucleotide sequence of SEQ ID NO: 76, and encodes a polypeptide which has a transmembrane domain, and can be localized in intracellular vesicles (for example, golgi bodies) or exosomes;
(h) a polynucleotide which consists of a nucleotide sequence obtained by deletion, substitution, addition or insertion of one or several nucleotides with respect to the nucleotide sequence of SEQ ID NO: 76, and encodes a polypeptide which has a transmembrane domain, and can be localized in intracellular vesicles (for example, golgi bodies) or exosomes;
(i) a polynucleotide which is a splicing variant of a polynucleotide consisting of the nucleotide sequence of SEQ ID NO: 76, and encodes a polypeptide which has a transmembrane domain, and can be localized in intracellular vesicles (for example, golgi bodies) or exosomes;
(j) a polynucleotide which is hybridized under stringent conditions to a complementary strand of a polynucleotide consisting of the nucleotide sequence of SEQ ID NO: 76, and encodes a polypeptide which has a transmembrane domain, and can be localized in intracellular vesicles (for example, golgi bodies) or exosomes;
(k) a polynucleotide which is a fragment of any of the polynucleotides (f) to (j), and encodes a polypeptide which has a transmembrane domain, and can be localized in intracellular vesicles (for example, golgi bodies) or exosomes;
(l) a polynucleotide encoding a polypeptide consisting of the amino acid sequence of SEQ ID NO: 88;
(m) a polynucleotide encoding a polypeptide which consists of an amino acid sequence having an identity of at least 80% with the amino acid sequence of SEQ ID NO: 88, has a transmembrane domain, and can be localized in intracellular vesicles (for example, golgi bodies) or exosomes;
(n) a polynucleotide encoding a polypeptide which consists of an amino acid sequence obtained by deletion, substitution, addition or insertion of one or several amino acids with respect to the amino acid sequence of SEQ ID NO: 88, has a transmembrane domain, and can be localized in intracellular vesicles (for example, golgi bodies) or exosomes; and
(o) a polynucleotide encoding a polypeptide which is a fragment of any of the polypeptides in (l) to (n), has a transmembrane domain, and can be localized in intracellular vesicles (for example, golgi bodies) or exosomes.

Specific examples of the polynucleotide encoding VAMP3 include the following:
(f) a polynucleotide consisting of the nucleotide sequence of SEQ ID NO: 77;
(g) a polynucleotide which consists of a nucleotide sequence having an identity of at least 80% with the nucleotide sequence of SEQ ID NO: 77, and encodes a polypeptide which has a transmembrane domain, and can be localized in intracellular vesicles (for example, golgi bodies) or exosomes;
(h) a polynucleotide which consists of a nucleotide sequence obtained by deletion, substitution, addition or insertion of one or several nucleotides with respect to the nucleotide sequence of SEQ ID NO: 77, and encodes a polypeptide which has a transmembrane domain, and can be localized in intracellular vesicles (for example, golgi bodies) or exosomes;
(j) a polynucleotide which is hybridized under stringent conditions to a complementary strand of a polynucleotide consisting of the nucleotide sequence of SEQ ID NO: 77, and encodes a polypeptide which has a transmembrane domain, and can be localized in intracellular vesicles (for example, golgi bodies) or exosomes;
(k) a polynucleotide which is a fragment of any of the polynucleotides (f) to (h) and (j), and encodes a polypeptide which has a transmembrane domain, and can be localized in intracellular vesicles (for example, golgi bodies) or exosomes;
(l) a polynucleotide encoding a polypeptide consisting of the amino acid sequence of SEQ ID NO: 89;
(m) a polynucleotide encoding a polypeptide which consists of an amino acid sequence having an identity of at least 80% with the amino acid sequence of SEQ ID NO: 89, has a transmembrane domain, and can be localized in intracellular vesicles (for example, golgi bodies) or exosomes;
(n) a polynucleotide encoding a polypeptide which consists of an amino acid sequence obtained by deletion, substitution, addition or insertion of one or several amino acids with respect to the amino acid sequence of SEQ ID NO: 89, has a transmembrane domain, and can be localized in intracellular vesicles (for example, golgi bodies) or exosomes; and
(o) a polynucleotide encoding a polypeptide which is a fragment of any of the polypeptides in (l) to (n), has a transmembrane domain, and can be localized in intracellular vesicles (for example, golgi bodies) or exosomes.

Specific examples of the polynucleotide encoding VAMP4 include the following:
(f) a polynucleotide consisting of the nucleotide sequence of SEQ ID NO: 78;
(g) a polynucleotide which consists of a nucleotide sequence having an identity of at least 80% with the nucleotide sequence of SEQ ID NO: 78, and encodes a polypeptide which has a transmembrane domain, and can be localized in intracellular vesicles (for example, golgi bodies or trans-golgi networks) or exosomes;
(h) a polynucleotide which consists of a nucleotide sequence obtained by deletion, substitution, addition or insertion of one or several nucleotides with respect to the nucleotide sequence of SEQ ID NO: 78, and encodes a polypeptide which has a transmembrane domain, and can be localized in intracellular vesicles (for example, golgi bodies or trans-golgi networks) or exosomes;
(i) a polynucleotide which is a splicing variant of a polynucleotide consisting of the nucleotide sequence of SEQ ID NO: 78, and encodes a polypeptide which has a transmembrane domain, and can be localized in intracellular vesicles (for example, golgi bodies or trans-golgi networks) or exosomes;
(j) a polynucleotide which is hybridized under stringent conditions to a complementary strand of a polynucleotide consisting of the nucleotide sequence of SEQ ID NO: 78, and encodes a polypeptide which has a transmembrane domain, and can be localized in intracellular vesicles (for example, golgi bodies or trans-golgi networks) or exosomes;
(k) a polynucleotide which is a fragment of any of the polynucleotides (f) to (j), and encodes a polypeptide which has a transmembrane domain, and can be localized in intracellular vesicles (for example, golgi bodies or trans-golgi networks) or exosomes;
(l) a polynucleotide encoding a polypeptide consisting of the amino acid sequence of SEQ ID NO: 90;
(m) a polynucleotide encoding a polypeptide which consists of an amino acid sequence having an identity of at least 80% with the amino acid sequence of SEQ ID NO: 90, has a transmembrane domain, and can be localized in intracellular vesicles (for example, golgi bodies or trans-golgi networks) or exosomes;
(n) a polynucleotide encoding a polypeptide which consists of an amino acid sequence obtained by deletion, substitution, addition or insertion of one or several amino acids with respect to the amino acid sequence of SEQ ID NO: 90, has a transmembrane domain, and can be localized in intracellular vesicles (for example, golgi bodies or trans-golgi networks) or exosomes; and
(o) a polynucleotide encoding a polypeptide which is a fragment of any of the polypeptides in (l) to (n), has a transmembrane domain, and can be localized in intracellular vesicles (for example, golgi bodies or trans-golgi networks) or exosomes.

Specific examples of the polynucleotide encoding VTI1B include the following:
(f) a polynucleotide consisting of the nucleotide sequence of SEQ ID NO: 79;
(g) a polynucleotide which consists of a nucleotide sequence having an identity of at least 80% with the nucleotide sequence of SEQ ID NO: 79, and encodes a polypeptide which has a transmembrane domain, and can be localized in intracellular vesicles (for example, early endosomes, late endosomes, lysosomes or recycling endosomes) or exosomes;
(h) a polynucleotide which consists of a nucleotide sequence obtained by deletion, substitution, addition or insertion of one or several nucleotides with respect to the nucleotide sequence of SEQ ID NO: 79, and encodes a polypeptide which has a transmembrane domain, and can be localized in intracellular vesicles (for example, early endosomes, late endosomes, lysosomes or recycling endosomes) or exosomes;
(j) a polynucleotide which is hybridized under stringent conditions to a complementary strand of a polynucleotide consisting of the nucleotide sequence of SEQ ID NO: 79, and encodes a polypeptide which has a transmembrane domain, and can be localized in intracellular vesicles (for example, early endosomes, late endosomes, lysosomes or recycling endosomes) or exosomes;
(k) a polynucleotide which is a fragment of any of the polynucleotides (f) to (h) and (j), and encodes a polypeptide which has a transmembrane domain, and can be localized in intracellular vesicles (for example, early endosomes, late endosomes, lysosomes or recycling endosomes) or exosomes;
(l) a polynucleotide encoding a polypeptide consisting of the amino acid sequence of SEQ ID NO: 91;
(m) a polynucleotide encoding a polypeptide which consists of an amino acid sequence having an identity of at least 80% with the amino acid sequence of SEQ ID NO: 91, has a transmembrane domain, and can be localized in intracellular vesicles (for example, early endosomes, late endosomes, lysosomes or recycling endosomes) or exosomes;
(n) a polynucleotide encoding a polypeptide which consists of an amino acid sequence obtained by deletion, substitution, addition or insertion of one or several amino acids with respect to the amino acid sequence of SEQ ID NO: 91, has a transmembrane domain, and can be localized in intracellular vesicles (for example, early endosomes, late endosomes, lysosomes or recycling endosomes) or exosomes; and
(o) a polynucleotide encoding a polypeptide which is a fragment of any of the polypeptides in (l) to (n), has a transmembrane domain, and can be localized in intracellular vesicles (for example, early endosomes, late endosomes, lysosomes or recycling endosomes) or exosomes.

Specific examples of the polynucleotide encoding BET1 include the following:
(f) a polynucleotide consisting of the nucleotide sequence of SEQ ID NO: 80;
(g) a polynucleotide which consists of a nucleotide sequence having an identity of at least 80% with the nucleotide sequence of SEQ ID NO: 80, and encodes a polypeptide which has a transmembrane domain, and can be localized in intracellular vesicles (for example, endoplasmic reticula, golgi bodies or cis-golgi networks) or exosomes;
(h) a polynucleotide which consists of a nucleotide sequence obtained by deletion, substitution, addition or insertion of one or several nucleotides with respect to the nucleotide sequence of SEQ ID NO: 80, and encodes a polypeptide which has a transmembrane domain, and can be localized in intracellular vesicles (for example, endoplasmic reticula, golgi bodies or cis-golgi networks) or exosomes;
(i) a polynucleotide which is a splicing variant of a polynucleotide consisting of the nucleotide sequence of SEQ ID NO: 80, and encodes a polypeptide which has a transmembrane domain, and can be localized in intracellular vesicles (for example, endoplasmic reticula, golgi bodies or cis-golgi networks) or exosomes;
(j) a polynucleotide which is hybridized under stringent conditions to a complementary strand of a polynucleotide consisting of the nucleotide sequence of SEQ ID NO: 80, and encodes a polypeptide which has a transmembrane domain, and can be localized in intracellular vesicles (for example, endoplasmic reticula, golgi bodies or cis-golgi networks) or exosomes;
(k) a polynucleotide which is a fragment of any of the polynucleotides (f) to (h) and (j), and encodes a polypeptide which has a transmembrane domain, and can be localized in intracellular vesicles (for example, endoplasmic reticula, golgi bodies or cis-golgi networks) or exosomes;
(l) a polynucleotide encoding a polypeptide consisting of the amino acid sequence of SEQ ID NO: 92;
(m) a polynucleotide encoding a polypeptide which consists of an amino acid sequence having an identity of at least 80% with the amino acid sequence of SEQ ID NO: 92, has a transmembrane domain, and can be localized in intracellular vesicles (for example, endoplasmic reticula, golgi bodies or cis-golgi networks) or exosomes;
(n) a polynucleotide encoding a polypeptide which consists of an amino acid sequence obtained by deletion, substitution, addition or insertion of one or several amino acids with respect to the amino acid sequence of SEQ ID NO: 92, has a transmembrane domain, and can be localized in intracellular vesicles (for example, endoplasmic reticula, golgi bodies or cis-golgi networks) or exosomes; and
(o) a polynucleotide encoding a polypeptide which is a fragment of any of the polypeptides in (l) to (n), has a transmembrane domain, and can be localized in intracellular vesicles (for example, endoplasmic reticula, golgi bodies or cis-golgi networks) or exosomes.

Specific examples of the polynucleotide encoding BET1L include the following:
(f) a polynucleotide consisting of the nucleotide sequence of SEQ ID NO: 81;
(g) a polynucleotide which consists of a nucleotide sequence having an identity of at least 80% with the nucleotide sequence of SEQ ID NO: 81, and encodes a polypeptide which has a transmembrane domain, and can be localized in intracellular vesicles (for example, golgi bodies or trans-golgi networks) or exosomes;
(h) a polynucleotide which consists of a nucleotide sequence obtained by deletion, substitution, addition or insertion of one or several nucleotides with respect to the nucleotide sequence of SEQ ID NO: 81, and encodes a polypeptide which has a transmembrane domain, and can be localized in intracellular vesicles (for example, golgi bodies or trans-golgi networks) or exosomes;
(i) a polynucleotide which is a splicing variant of a polynucleotide consisting of the nucleotide sequence of SEQ ID NO: 81, and encodes a polypeptide which has a transmembrane domain, and can be localized in intracellular vesicles (for example, golgi bodies or trans-golgi networks) or exosomes;
(j) a polynucleotide which is hybridized under stringent conditions to a complementary strand of a polynucleotide consisting of the nucleotide sequence of SEQ ID NO: 81, and encodes a polypeptide which has a transmembrane domain, and can be localized in intracellular vesicles (for example, golgi bodies or trans-golgi networks) or exosomes;
(k) a polynucleotide which is a fragment of any of the polynucleotides (f) to (j), and encodes a polypeptide which has a transmembrane domain, and can be localized in intracellular vesicles (for example, golgi bodies or trans-golgi networks) or exosomes;
(l) a polynucleotide encoding a polypeptide consisting of the amino acid sequence of SEQ ID NO: 93;
(m) a polynucleotide encoding a polypeptide which consists of an amino acid sequence having an identity of at least 80% with the amino acid sequence of SEQ ID NO: 93, has a transmembrane domain, and can be localized in intracellular vesicles (for example, golgi bodies or trans-golgi networks) or exosomes;
(n) a polynucleotide encoding a polypeptide which consists of an amino acid sequence obtained by deletion, substitution, addition or insertion of one or several amino acids with respect to the amino acid sequence of SEQ ID NO: 93, has a transmembrane domain, and can be localized in intracellular vesicles (for example, golgi bodies or trans-golgi networks) or exosomes; and (o) a polynucleotide encoding a polypeptide which is a fragment of any of the polypeptides in (l) to (n), has a transmembrane domain, and can be localized in intracellular vesicles (for example, golgi bodies or trans-golgi networks) or exosomes.

Specific examples of the polynucleotide encoding USE1 include the following:
(f) a polynucleotide consisting of the nucleotide sequence of SEQ ID NO: 82;
(g) a polynucleotide which consists of a nucleotide sequence having an identity of at least 80% with the nucleotide sequence of SEQ ID NO: 82, and encodes a polypeptide which has a transmembrane domain, and can be localized in intracellular vesicles (for example, endoplasmic reticula) or exosomes;
(h) a polynucleotide which consists of a nucleotide sequence obtained by deletion, substitution, addition or insertion of one or several nucleotides with respect to the nucleotide sequence of SEQ ID NO: 82, and encodes a polypeptide which has a transmembrane domain, and can be localized in intracellular vesicles (for example, endoplasmic reticula) or exosomes;
(j) a polynucleotide which is hybridized under stringent conditions to a complementary strand of a polynucleotide consisting of the nucleotide sequence of SEQ ID NO: 82, and encodes a polypeptide which has a transmembrane domain, and can be localized in intracellular vesicles (for example, endoplasmic reticula) or exosomes;
(k) a polynucleotide which is a fragment of any of the polynucleotides (f) to (h) and (j), and encodes a polypeptide which has a transmembrane domain, and can be localized in intracellular vesicles (for example, endoplasmic reticula) or exosomes;
(l) a polynucleotide encoding a polypeptide consisting of the amino acid sequence of SEQ ID NO: 94;
(m) a polynucleotide encoding a polypeptide which consists of an amino acid sequence having an identity of at least 80% with the amino acid sequence of SEQ ID NO: 94, has a transmembrane domain, and can be localized in intracellular vesicles (for example, endoplasmic reticula) or exosomes;
(n) a polynucleotide encoding a polypeptide which consists of an amino acid sequence obtained by deletion, substitution, addition or insertion of one or several amino acids with respect to the amino acid sequence of SEQ ID NO: 94, has a transmembrane domain, and can be localized in intracellular vesicles (for example, endoplasmic reticula) or exosomes; and
(o) a polynucleotide encoding a polypeptide which is a fragment of any of the polypeptides in (l) to (n), has a transmembrane domain, and can be localized in intracellular vesicles (for example, endoplasmic reticula) or exosomes.

The method for acquiring the polynucleotide encoding a SNARE protein according to the present invention is not limited, and the polynucleotide can be obtained by an ordinary chemical synthesis method or genetic engineering method. For example, a polynucleotide encoding a SNARE protein can be artificially synthesized on the basis of any of the nucleotide sequences SEQ ID NOS: 1 to 9 and 71 to 82. For the artificial synthesis, for example, a commercially available DNA synthesis service provided from GenScript or the like can be used. Alternatively, for example, any of the nucleotide sequences SEQ ID NOS: 1 to 9 and 71 to 82 can be cloned from a human-derived sample by a method described in Molecular Cloning-A LABORATORY MANUAL THIRD EDITION (Joseph Sambrook, David W. Russell, Cold Spring Harbor Laboratory Press, 2001).

The polynucleotide encoding a SNARE protein according to the present invention can also be produced by, for example, introducing a mutation into DNA consisting of any of the nucleotide sequences of SEQ ID NOS: 1 to 9 and 71 to 82. Examples of the method for introducing a mutation include ultraviolet irradiation and site-directed mutagenesis methods. Examples of the site-directed mutagenesis method include a method utilizing splicing overlap extension (SOE) PCR (Horton et al., Gene, 77, 61-68, 1989), an ODA method (Hashimoto-Gotoh et al., Gene, 152, 271-276, 1995), and a Kunkel method (Kunkel, T. A., Proc. Natl. Acad. Sci. USA, 1985, 82, 488). Alternatively, a commercially available kit for site-directed mutagenesis such as Site-Directed Mutagenesis System Mutan-SuperExpress Km Kit (Takara Bio Inc.), Transformer^{™} Site-Directed Mutagenesis Kit (Clonetech Laboratories, Inc.) or KOD-Plus-Mutagenesis Kit (TOYOBO CO., LTD.) can be used. From DNAs into which a mutation has been introduced, DNA which has a transmembrane domain and can be localized in intracellular vesicles is selected, thereby enabling acquirement of a polynucleotide encoding a SNARE protein according to the present invention. Whether the polypeptide encoded by DNA into which a mutation has been introduced has a transmembrane domain or not can be determined by, for example, use of a prediction tool such as SOSUI (Hirokawa et al., Bioinformatics, 14(4): 378-379, 1998) or TMHMM (Krough et al., J Mol Biol. 305(3): 567-580, 2001) on the basis of the amino acid sequence of the polypeptide encoded by DNA.

The method for deletion, substitution, addition or insertion of a nucleotide in a nucleotide sequence is described in, for example, Dieffenbach et al (Cold Spring Harbar Laboratory Press, New York, 581-621, 1995).

The polynucleotide encoding a SNARE protein according to the present invention can also be obtained by, for example, subjecting DNA consisting of any of the nucleotide sequences of SEQ ID NOS: 1 to 9 and 71 to 82 to genome editing using an artificial DNA cleavage enzyme (artificial DNA nuclease or programmable nuclease).

In the present invention, any allergen may be used. In the present invention, the allergen means a protein which enters a living organism from the outside through inhalation, insertion, intake or contact and triggers a hypersensitive reaction or an allergic reaction, or a peptide derived therefrom. The allergen is not limited, and examples thereof include proteins contained in pollens of gramineous plants (reed grass, timothy grass, meadow foxtail, orchard grass, Bermuda grass, wheat, creeping bent grass, Japanese paspalum, Jhonson grass, Kentucky bluegrass, vernal grass, meadow fescue, rye grass and the like); pollens of weeds (golden rod, stinging nettle, giant ragweed, Japanese hop, goosefoot, dandelion, wormwood, sheep's sorrel, common ragweed, western ragweed, ox-eye daisy, ribgrass, mugwort and the like); pollens of trees (acacia, olive, maple, walnut, mulberry, konara oak, Japanese white birch, cedar elm, Japanese alder, Japanese cedar, hinoki cypress, juniper, Japanese beech, pine, willow and the like); fungi or bacteria (aspergillus, Alternaria, Staphylococcus aureus enterotoxin A, Staphylococcus aureus enterotoxin B, Candida, Cladosporium, Trichophyton, Pityrosporum, Penicillium, Helminthosporium, Malassezia, Mucor and the like); animal skins (duck feathers, cat dander, dog dander, cow dander, horse dander, rabbit dander, hamster epithelium, guinea pig epithelium, sheep epithelium, pig epithelium, goat epithelium, chicken feathers, goose feathers, budgerigar feathers, budgerigar droppings, mice, rats and the like); insects (paper wasps, moths, cockroaches, hornets, honeybees, aedes, chironomids (adults) and the like); parasitic insects (anisakis, ascaris and the like); mites (Acarus siro, Tyrophagus putrescentiae, Dermatophagoides farinae, Lepidoglyphus destructor, Dermatophagoides pteronyssinus and the like); foods (egg, milk, wheat, buckwheat, peanut, shrimp, crab, almond, abalone, squid, salmon roe, orange, cashewnut, kiwi fruit, beef, walnut, sesame, salmon, mackerel, soybean, chicken meat, banana, pork, matsutake mushroom, peach, yam, apple, gelatin and the like); human insulins; and the like, or peptides derived therefrom. In particular, the allergen is preferably a protein contained in food, or a peptide derived therefrom, more preferably a protein contained in egg, or a peptide derived therefrom.

The method for acquiring the polynucleotide encoding an allergen according to the present invention is not limited, and the polynucleotide can be obtained by an ordinary chemical synthesis method or genetic engineering method as in the case of the acquirement of a polynucleotide encoding a SNARE protein as above. The polynucleotide encoding an allergen may be a polynucleotide encoding the full length of the allergen, or may be a polynucleotide encoding a partial polypeptide of the allergen as long as it can function as an allergen. A plurality of polynucleotides each encoding one allergen may be expressibly linked consecutively, or a polynucleotide encoding two or more allergens may be expressibly linked. One or more, preferably five or less, more preferably three or less, further more preferably two or less types of allergens are used for the nucleic acid structure of the present invention. Five types, four types, three types, two types or one type of allergen can be used for the nucleic acid structure of the present invention.

The nucleic acid structure of the present invention is preferably a nucleic acid structure in which a polynucleotide encoding an allergen is expressibly linked downstream of a polynucleotide encoding a SNARE protein, more preferably a nucleic acid structure in which a polynucleotide encoding an allergen is expressibly linked downstream of a polynucleotide encoding a SNARE protein via a polynucleotide encoding a linker and/or a polynucleotide encoding a proprotein convertase recognition sequence, further more preferably a nucleic acid structure in which a polynucleotide encoding an allergen is expressibly linked downstream of a polynucleotide encoding a SNARE protein via a polynucleotide encoding a linker and a polynucleotide encoding a proprotein convertase recognition sequence. When the nucleic acid structure of the present invention contains a polynucleotide encoding a linker and a polynucleotide encoding a proprotein convertase recognition sequence, the order of linkage of these polynucleotides is not limited, and the polynucleotide encoding a proprotein convertase recognition sequence may be linked downstream of the polynucleotide encoding a linker, or the polynucleotide encoding a linker may be linked downstream of the polynucleotide encoding a proprotein convertase recognition sequence. A sequence having about 1 to 5 repetitions of a proprotein convertase recognition sequence may be linked, or a polynucleotide encoding a proprotein convertase recognition sequence may be linked so as to be sandwiched between a plurality of polynucleotides encoding a linker. Any polypeptide other than the polynucleotide encoding a linker and the polynucleotide encoding a proprotein convertase recognition sequence may be present between the polynucleotide encoding a SNARE protein and the polynucleotide encoding an antigen as long as the expression of both the polynucleotides is not impaired.

In the present invention, the term "linker" refers to a peptide linker which connects two polypeptides. The linker is not limited as long as it allows the SNARE protein and the allergen to normally function. The linker has a length of preferably 3 or more amino acid residues, more preferably 4 or more amino acid residues, further more preferably 5 or more amino acid residues, and preferably 30 or less amino acid residues, more preferably 25 or less amino acid residues, further more preferably 20 or less amino acid residues. The linker has a length of preferably from 3 to 30 amino acid residues, more preferably from 4 to 25 amino acid residues, further more preferably from 5 to 20 amino acid residues. Examples of the linker include linkers having G, GS, GGS, GGGS (SEQ ID NO: 96), GGGGS (SEQ ID NO: 97), EAAAK (SEQ ID NO: 98) or XP as a constituent element. Herein, X represents any amino acid residue. Specific examples thereof include linkers consisting of a sequence having 1 to 5, preferably 3 or 4 repetitions of any of the constituent elements, and preferred examples include a linker consisting of 3 repetitions of GGGGS (SEQ ID NO: 20). The amino acid residue S may be added before the repeating sequence when the constituent element of the sequence is GGGGS, or the amino acid residue A may be added before and after the repeating sequence when the constituent element of the sequence is EAAAK.

In the present invention, the term "proprotein convertase recognition sequence" refers to an amino acid sequence which is recognized by a proprotein convertase, that is a serine protease, which converts a proprotein into a physiologically active protein or peptide in a golgi body or the like. Specifically, the proprotein convertase recognition sequence is an amino acid sequence of X-Arg-X-(Arg/Lys)-Arg (for example, SEQ ID NO: 22). Herein, X represents any amino acid residue. Examples of the proprotein convertase include furin, PC2, PC4, PC5/6, PC7, and PACE4, and all these enzymes are known to well recognize an Arg-X-(Arg/Lys)-Arg motif (Remacle AG, et al. Journal of Biological Chemistry 2008, 283(30): 20897-20906). Thus, the proprotein convertase recognition sequence can be Arg-X-(Arg/Lys)-Arg. Herein, X represents any amino acid residue. The proprotein convertase recognizes the above-described amino acid sequence, and cleaves the C-terminal side of arginine residue on the C-terminal. In the present invention, it is preferable that the proprotein convertase be furin, where the furin recognition sequence is identical to the above-described proprotein convertase recognition sequence.

The method for acquiring a polynucleotide encoding a linker or a polynucleotide encoding a proprotein convertase recognition sequence is not limited, and the polynucleotide can be obtained by an ordinary chemical synthesis method or genetic engineering method.

If necessary, the polynucleotide encoding a SNARE protein, the polynucleotide encoding an allergen, the polynucleotide encoding a linker or the polynucleotide encoding a proprotein convertase recognition sequence, which is contained in the nucleic acid structure of the present invention, may be codon-optimized in a manner suitable for the type of a subject to which the nucleic acid structure is administered. The information of codons used by various living organisms is available from Codon Usage Database ([www.kazusa.or.jp/codon/].

In a preferred example, the nucleic acid structure of the present invention is an expression cassette, and contains a control region for controlling expression of the polynucleotide encoding a SNARE protein and the polynucleotide encoding an allergen. In the expression cassette, the polynucleotide encoding a SNARE protein and the polynucleotide encoding an allergen are operably linked to the control region. Examples of the control region include promoters, terminators, and enhancers. Preferably, the expression cassette contains a promoter linked upstream of the polynucleotide encoding a SNARE protein and the polynucleotide encoding an allergen.

The nucleic acid structure of the present invention may have a restriction enzyme recognition site at one end or both ends thereof. The nucleic acid structure of the present invention can be introduced into a vector using the restriction enzyme recognition site. For example, by cleaving a vector with a restriction enzyme, and adding thereto the nucleic acid structure of the present invention having a restriction enzyme recognition site at the end, the nucleic acid structure can be introduced into the vector.

The type of vector is not limited, and any vector may be used such as a plasmid vector, a phage, a phargemid, a cosmid or a virus vector. In an example, the vector into which the nucleic acid structure of the present invention is to be incorporate can be an expression vector, but is not required to be an expression vector when the nucleic acid structure to be incorporated is an expression cassette. In an example, the nucleic acid structure of the present invention is an expression cassette containing a control region, and is incorporated into any vector to construct an expression vector. In another example, by incorporating the nucleic acid structure of the present invention into an expression vector containing a control region, an expression cassette according to the present invention is constructed on the expression vector.

In a preferred example, the nucleic acid structure of the present invention is a plasmid vector. Examples of the plasmid vector include, but are not limited to, pVAX1. Here, in a plasmid vector, a drug-resistant gene is generally incorporated in the sequence for the purpose of selectively retaining the plasmid vector during culturing of bacteria, but since there is a risk of propagation of the gene to bacterial flora or activation and expression of the gene via a mammal promoter, a plasmid vector free from the relevant gene sequence is more preferable.

In a preferred example, the nucleic acid structure of the present invention is a virus vector. Examples of the virus vector include, but are not limited to, adenovirus vectors, adeno-associated virus vectors, lentivirus vectors, retrovirus vectors, Sendai virus vectors, and herpesvirus vectors.

In a preferred example, the nucleic acid structure of the present invention is a mRNA structure. The nucleic acid structure of the present invention can be obtained as mRNA by, for example, using a commercially available *in vitro* transcription kit such as HiScribe T7 ARCA mRNA Kit (New England Biolabs Inc.), T7 mScript^{™} Standard mRNA production System (CELLSCRIPT) or HighYield T7 ARCA mRNA Synthesis Kit (Gena Bioscience) with DNA as a template, where the DNA contains a polynucleotide encoding a SNARE protein and a polynucleotide encoding an allergen.

As shown in Examples described later, the nucleic acid structure according to the present invention containing a polynucleotide encoding a SNARE protein and a polynucleotide encoding an allergen has higher immunogenicity, and can induce or enhance an allergen-specific Th1-type immune response and induce or enhance an allergen-specific cellular immune response, as compared to a nucleic acid structure containing a polynucleotide encoding an allergen (hereinafter, referred to as a control). This may be because in the antigen presentation process where a nucleic acid structure incorporated into cells is translated or transcribed and translated, thus expressed as an antigenic protein, and transported to intracellular vesicles, and a part of the antigen binds to a major histocompatibility complex (MHC) molecule, followed by transportation to cell surfaces, the nucleic acid structure of the present invention allows the allergen protein to be expressed as a fusion polypeptide of a SNARE protein and an allergen to attempt the targeting of the allergen protein to intracellular vesicles, so that the probability of association with the MHC molecule increases to improve the efficiency of antigen presentation. In addition, exosomes containing an antigen have been reported to enhance the antigenspecific Th1-type immune response *in vivo* (Qazi KR, et al. Blood. 2009, 113(12): 2673-83.). Thus, there may be a possibility that the allergen protein is expressed as a fusion polypeptide of a SNARE protein and an allergen to attempt the targeting of the allergen protein to exosomes, resulting in improvement of immunogenicity.

As shown in Examples described later, in an allergy model, the nucleic acid structure of the present invention can ameliorate a decrease in body temperature due to anaphylaxis, and reduce the blood concentration of allergen-specific IgE antibody, as compared to a control. These results indicate that the nucleic acid structure of the present invention has an excellent allergy suppressing effect. Further, it is known that in conventional allergy immunotherapy, a part of an exogenously ingested allergen is released into the blood, and caught by an allergen-specific IgE antibody, thus causing a side effect which falls under the category of type I allergies such as anaphylaxis, whereas as shown in Examples described later, in the case of the nucleic acid structure of the present invention, release of an allergen into the blood after administration is suppressed. These results indicate that the nucleic acid structure of the present invention may be capable of reducing the risk of anaphylaxis.

The amount of production of cytokines such as IFNγ and IL-4 which can be an index for determining whether the immune response is of Th1 type or Th2 type, or whether the immune response is a cellular immune response or a humoral immune response can be measured by a heretofore known method. Examples of the method include ELISA (enzyme-linked immunosorbent assay), ELISPOT (enzyme-linked immunosorbent spot) assay, immunohistostaining, in situ hybridization, RT-PCR, micro-arraying, and flow cytometry. Commercially available reagents and kits for the measurement of the production of cytokines such as an ELISPOT assay kit (Cellular Technology Limited) used in Examples described later may be used.

The amount of production of each IgG subclass which can be another index for determining whether the immune response is of Th1 type or Th2 type, or whether the immune response is a cellular immune response or a humoral immune response can be measured by a heretofore known method. Examples of the method include ELISA, and immunoturbidimetry.

The Th1-type immune response and the cellular immune response can be evaluated using the amount of production of cytokines and/or the amount of production of an IgG subclass as indices. For example, when the amount of production of IFNγ or the ratio of the amount of production of IFNγ to the amount of production of IL-4 (IFNy/IL-4) increases, it can be evaluated that the Th1-type immune response and the cellular immune response are induced or enhanced, whereas when the amount of production of IFNγ or the IFNγ/IL-4 decreases, it can be evaluated that the Th1-type immune response and the cellular immune response are reduced. Alternatively, the amount of production of IgG2a and/or the ratio of the amount of production of IgG2a to the amount of production of IgG1 (IgG2a/IgG1) increases, it can be evaluated that the Th1-type immune response and the cellular immune response are induced or enhanced, and when the amount of production of IgG2a and/or the IgG2a/IgG1 ratio decreases, it can be evaluated that the Th1-type immune response and the cellular immune response are reduced.

Therefore, the nucleic acid structure of the present invention can be an allergen-specific IFNγ production enhancing agent, an allergen-specific IgG2a production enhancing agent, an allergen-specific Th1-type immune response inducing or enhancing agent or an allergen-specific cellular immune response inducing or enhancing agent (hereinafter, referred to as "a cellular immune response inducing or enhancing agent or the like"), and the nucleic acid structure can be used for producing a cellular immune response inducing or enhancing agent or the like.

The nucleic acid structure of the present invention can be used for enhancing allergen-specific IFNγ production, enhancing allergen-specific IgG2a production, inducing or enhancing an allergen-specific Th1-type immune response, or inducing or enhancing an allergen-specific cellular immune response. The use can be use in administration to a human or a non-human animal, or in a specimen derived therefrom, and may be therapeutic use or non-therapeutic use. The term "non-therapeutic" refers to a concept of not including a medical practice, that is, a concept of not including a method of operating, treating or diagnosing a human, more specifically, a concept of not including a method of performing an operation, treatment or diagnosis on a human by a doctor or a person instructed by a doctor.

The cellular immune response inducing or enhancing agent or the like of the present invention is itself a pharmaceutical product or a quasi-pharmaceutical product for enhancing allergen-specific IFNγ production, enhancing allergen-specific IgG2a production, inducing or enhancing an allergen-specific Th1-type immune response, or inducing or enhancing an allergen-specific cellular immune response, and can be a material or a preparation which is used by being blended in the pharmaceutical product or quasi-pharmaceutical product.

When the cellular immune response inducing or enhancing agent or the like of the present invention is used as a pharmaceutical product (including a quasi-pharmaceutical product), the pharmaceutical product can be administered in any dosage form. Examples of the dosage form include oral administration such as tablets, capsules, granules, powders and syrups, and parental administration such as injection preparations, suppositories, inhalants, transdermal patches and external preparations. Parenteral administration is preferable, and parenteral administration with an injection preparation is more preferable.

Such pharmaceutical preparations of various dosage forms can be prepared by appropriately combining the nucleic acid structure of the present invention with other pharmaceutically acceptable components such as an excipient, a binder, an extender, a disintegrant, a diluent, a thickener, an emulsifier, a lubricant, a dispersant, a film forming agent, a surfactant, a film forming agent, an osmotic pressure adjuster, a buffer, a pH adjuster, a preservative, a stabilizer, an antioxidant, a colorant, a taste improving agent, an odor improving agent and a flavor.

The content of the nucleic acid structure of the present invention in the pharmaceutical product (including a quasi-pharmaceutical product) is not limited and is appropriately selected from a broad range because it varies depending on an allergen of interest, a subject to be administrated and a route of administration. As an example, the content of the nucleic acid structure can be in the range of from 0.00001 to 100% of the total amount of the composition on a mass basis.

The dosage amount of the cellular immune response inducing or enhancing agent or the like of the present invention can vary depending on the type, the body weight, the sex, the age and the condition of a subject, or other factors. The dose, the route and the administration interval can be appropriately determined by those skilled in the art. For example, the dosage amount is determined within the range of from 1 ng to 10 mg a day per adult (body weight: 60 kg) in terms of the nucleic acid structure of the present invention. When the nucleic acid structure is a virus vector, an example of the dosage amount can be from 10 to 1 × 10¹⁵ virus particles a day per adult (body weight: 60 kg).

The cellular immune response inducing or enhancing agent or the like of the present invention can be administered to either a human or a non-human animal. Examples of the non-human animal include non-human mammals, and examples of the non-human mammal include apes, other primates, mice, rats, horses, cattle, pigs, sheep, dogs, cats, hamsters, and companion animals. Preferably, the cellular immune response inducing or enhancing agent or the like of the present invention is administered to a human. More preferably, the cellular immune response inducing or enhancing agent or the like of the present invention is administered to an allergy patient or a human suspected to have an allergy.

The nucleic acid structure of the present invention can be nucleic acid vaccine, and the nucleic acid structure can be used for producing nucleic acid vaccine.

Further, the nucleic acid structure of the present invention can be used for inducing or enhancing an allergen-specific Th1-type immune response and/or inducing or enhancing an allergen-specific cellular immune response. Here, the use can be use in administration to a human or a non-human animal, or in a specimen derived therefrom, and may be therapeutic use or non-therapeutic use.

The nucleic acid vaccine of the present invention is itself a pharmaceutical product for inducing or enhancing an allergen-specific Th1-type immune response and/or inducing or enhancing an allergen-specific cellular immune response, and can be a material of a preparation which is used by being blended in the pharmaceutical product.

In an embodiment, the nucleic acid vaccine is DNA vaccine. The DNA vaccine contains the nucleic acid structure of the present invention which is a plasmid vector. The plasmid vector is not limited, and examples thereof include pVAX1 vectors. In particular, plasmid vectors free from a drug-resistant gene are preferable.

In another embodiment, the nucleic acid vaccine is mRNA vaccine. The mRNA vaccine contains the nucleic acid structure of the present invention which is mRNA, and the nucleic acid structure may be mRNA treated to add a cap structure or poly A thereto and/or mRNA in which some bases are modified (for example, uridine is replaced with pseudouridine or 1-methylpseudouridine), for stabilization of mRNA, improvement of translation efficiency or prevention of an excessive immune response. The mRNA vaccine may be self-amplifying RNA containing he nucleic acid structure of the present invention, a sequence of a virus-derived RNA-dependent RNA polymerase (RdRP) complex and an origin of replication thereof (5'CSE, 3'CSE), or trans-amplifying RNA obtained by mixing RNA containing the nucleic acid structure of the present invention and an origin of replication of a RdRP complex with mRNA containing a sequence of RdRP complex. Preferably, the mRNA vaccine further contains a structure engaged in drug delivery, such as liposomes or lipid nanoparticles with a lipid as a constituent component or polymer nanoparticles such as PLGA nanoparticles with a high-molecule polymer as a constituent component, as a carrier for stabilization and delivery of mRNA. More preferably, the mRNA is encapsulated in the structure.

In still another embodiment, the nucleic acid vaccine is virus vector vaccine. The virus vector vaccine contains the nucleic acid structure of the present invention which is a virus vector. The virus vector is not limited, and examples thereof include adenovirus vectors, adeno-associated virus vectors, lentivirus vectors, retrovirus vectors, Sendai virus vectors, and herpesvirus vectors.

The nucleic acid vaccine of the present invention contains a pharmaceutically acceptable carrier as appropriate in addition to the nucleic acid structure, and can be formulated into a predetermined form.

Here, examples of the carrier include carriers which are commonly used for production of vaccine, and specifically, a buffer, an emulsifier, a preserving agent (for example, thimerosal), a tonicity agent, a pH adjuster, a thickening agent, an adjuvant, an immune stimulant or the like is exemplified. The adjuvant is a substance which enhances an immune response to an antigen when administered together with the antigen. The nucleic acid vaccine of the present invention can itself function as an adjuvant, thus does not necessarily need addition of an adjuvant, and may be a composition free from an adjuvant.

The nucleic acid vaccine of the present invention is preferably in a liquid form, and is appropriately formulated so as to match an intended route of administration. The route of administration is classified into oral administration and parenteral administration. Examples thereof include intramuscular administration, intradermal administration, subcutaneous administration, transdermal administration, intranasal administration, sublingual administration, oral administration, and inhalation, and intramuscular administration, intradermal administration or subcutaneous administration is preferable. Examples of the dosage form of the preparation for injection administration include solutions, emulsions, watersoluble or hydrophobic suspensions, and dry powder which is used by being dissolved or suspended by addition of a liquid.

The content of the nucleic acid structure of the present invention in the nucleic acid vaccine of the present invention is not limited and is appropriately selected from a broad range because it varies depending on an allergen of interest, a subject to be administrated and a route of administration. As an example, the content of the nucleic acid structure can be in the range of from 0.00001 to 100% of the total amount of the nucleic acid vaccine on a mass basis.

The dosage amount of the nucleic acid vaccine of the present invention can vary depending on the type, the body weight, the sex, the age and the condition of a subject, or other factors. The dose, the route and the administration interval can be appropriately determined by those skilled in the art. For example, the dosage amount is determined within the range of from 1 ng to 10 mg per dosage unit in terms of the nucleic acid structure of the present invention. When the nucleic acid structure is a virus vector, an example of the dosage amount can be from 10 to 1 × 10¹⁵ virus particles per dosage unit.

The nucleic acid vaccine of the present invention can be administered to either a human or a non-human animal. Examples of the non-human animal are as described above. Preferably, the nucleic acid vaccine of the present invention is administered to a human. More preferably, the nucleic acid vaccine of the present invention is administered to an allergy patient or a human suspected to have an allergy.

The number of times of administration of the nucleic acid vaccine of the present invention may be appropriately set depending on a use, and is at least once, and may be twice or more from the viewpoint of effectiveness. The additional administration is sometimes referred to booster immunization, which enables exhibition of more effective protection against infection or a higher therapeutic effect. The interval of the booster immunization is recommended to be at least 1 week, and is preferably 1 to 4 weeks.

The nucleic acid vaccine can be applied to a variety of allergens by changing the nucleic acid encoding the allergen, and can be produced at low cost and a high rate due to the nature of the nucleic acid.

In a preferred embodiment, the nucleic acid vaccine of the present invention is a vaccine for prevention or treatment of an allergy, preferably a vaccine for allergy immunotherapy. Specifically, the vaccine for prevention or treatment of an allergy contains the nucleic acid structure according to the present invention containing a polynucleotide encoding a SNARE protein and a polynucleotide encoding an allergen. The vaccine for prevention or treatment of an allergy may be directly administered to a living organism. Such a vaccine for prevention or treatment of an allergy may be capable of inducing or enhancing an allergen-specific Th1-type immune response and cellular immune response to exhibit a suppressing effect on an allergic response caused by excessive activation of a Th2-type immune response.

As exemplary embodiments of the present invention, the following substances, production methods, uses, methods and the like are further disclosed herein. However, the present invention is not limited to these embodiments.
[1] A nucleic acid structure comprising a polynucleotide encoding a SNARE protein and a polynucleotide encoding an allergen.
[2] The nucleic acid structure according to [1], wherein the polynucleotide encoding an allergen is linked downstream side of the polynucleotide encoding a SNARE protein.
[3] The nucleic acid structure according to [1] or [2], wherein the polynucleotide encoding a SNARE protein and the polynucleotide encoding an allergen are linked via a polynucleotide encoding a linker and/or a polynucleotide encoding a proprotein convertase recognition sequence, preferably a polynucleotide encoding a linker and a polynucleotide encoding a proprotein convertase recognition sequence.
[4] The nucleic acid structure according to [3], wherein the proprotein convertase recognition sequence is an amino acid sequence consisting of Arg-X-(Arg/Lys)-Arg (X represents any amino acid residue), preferably an amino acid sequence consisting of X-Arg-X-(Arg/Lys)-Arg (X represents any amino acid residue), more preferably an amino acid sequence of SEQ ID NO: 22.
[5] The nucleic acid structure according to any one of [1] to [4], which is a plasmid vector, mRNA or a virus vector.
[6] The nucleic acid structure according to any one of [1] to [5], wherein the SNARE protein is preferably a mammal SNARE protein, more preferably a human SNARE protein.
[7] The nucleic acid structure according to any one of [1] to [6], wherein the polynucleotide encoding a SNARE protein is preferably any one selected from the group consisting of polynucleotides consisting of the nucleotide sequences of SEQ ID NOS: 1 to 9 and 71 to 82, and polynucleotides functionally equivalent thereto, more preferably any one polynucleotide consisting of the nucleotide sequences of SEQ ID NOS: 1 to 9 and 71 to 82.
[8] The nucleic acid structure according to any one of [1] to [7], wherein the SNARE protein is preferably any one selected from the group consisting of VAMP7, GOSR2, STX10, STX18, BNIP1, STX7, VTI1A, STX16, STX5, GOSR1, STX8, STX12, VAMP8, SEC22B, STX6, VAMP3, VAMP4, VTI1B, BET1, BET1L and USE1, more preferably any one selected from the group consisting of VAMP7, GOSR2, STX10, STX18, BNIP1, STX7, VTI1A, STX16, STX5, GOSR1, STX8, STX12, VAMP8 and SEC22B, further more preferably any one selected from the group consisting of VAMP7, GOSR2, STX10, STX18, BNIP1, STX7, VTI1A, STX16, STX5 and GOSR1, further more preferably any one selected from the group consisting of VAMP7, GOSR2, STX10, STX18, BNIP1, STX7, VTI1A and GOSR1, further more preferably any one selected from the group consisting of VAMP7, GOSR2, STX10 and GOSR1, further more preferably VAMP7.
[9] A pharmaceutical composition comprising the nucleic acid structure according to any one of [1] to [8].
[10] A nucleic acid vaccine comprising, as an active component, the nucleic acid structure according to any one of [1] to [8].
[11] The nucleic acid vaccine according to [10], which induces or enhances an allergen-specific Th1-type immune response.
[12] The nucleic acid vaccine according to [10], which induces or enhances an allergen-specific cellular immune response.
[13] The nucleic acid vaccine according to any one of [10] to [12], for use in prevention or treatment of an allergy.
[14] The nucleic acid vaccine according to any one of [10] to [13], wherein the nucleic acid vaccine preferably comprises from 0.00001 to 100 mass% of the nucleic acid structure.
[15] Use of the nucleic acid structure according to any one of [1] to [8] for producing nucleic acid vaccine.
[16] The use according to [15], wherein the nucleic acid vaccine is a vaccine for prevention or treatment of an allergy.
[17] The use according to [15] or [16], wherein the nucleic acid vaccine preferably comprises from 0.00001 to 100 mass% of the nucleic acid structure.
[18] Use of the nucleic acid structure according to any one of [1] to [8], for inducing or enhancing an allergen-specific Th1-type immune response and/or inducing or enhancing an allergen-specific cellular immune response.
[19] The nucleic acid structure according to any one of [1] to [8], for use in induction or enhancement of an allergen-specific Th1-type immune response and/or induction or enhancement of an allergen-specific cellular immune response.
[20] A method for inducing or enhancing an allergen-specific Th1-type immune response and/or inducing or enhancing an allergen-specific cellular immune response, the method comprising administering an effective amount of the nucleic acid structure according to any one of [1] to [8] to a subject in need thereof.
[21] Use of the nucleic acid vaccine according to any one of [10] to [14], for inducing or enhancing an allergen-specific Th1-type immune response and/or inducing or enhancing an allergen-specific cellular immune response.
[22] The nucleic acid vaccine according to any one of [10] to [14], for use in induction or enhancement of an allergen-specific Th1-type immune response and/or induction or enhancement of an allergen-specific cellular immune response.
[23] A method for inducing or enhancing an allergen-specific Th1-type immune response and/or inducing or enhancing an allergen-specific cellular immune response, the method comprising administering an effective amount of the nucleic acid vaccine according to any one of [10] to [14] to a subject in need thereof.
[24] A method for preventing or treating an allergy, the method comprising administering an effective amount of the nucleic acid vaccine according to [13] to a subject in need thereof.
[25] The method according to [23] or [24], wherein a dosage amount of the nucleic acid vaccine is preferably from 1 ng to 10 mg per dosage unit in terms of the nucleic acid structure when the nucleic acid vaccine is DNA vaccine or mRNA vaccine, and the dosage amount of the nucleic acid vaccine is preferably from 10 to 1 × 10¹⁵ virus particles per dosage unit when the nucleic acid vaccine is virus vector vaccine.
[26] An allergen-specific IFNγ production enhancing agent comprising, as an active component, the nucleic acid structure according to any one of [1] to [8].
[27] An allergen-specific IgG2a production enhancing agent comprising, as an active component, the nucleic acid structure according to any one of [1] to [8].
[28] An allergen-specific Th1-type immune response inducing or enhancing agent comprising, as an active component, the nucleic acid structure according to any one of [1] to [8].
[29] An allergen-specific cellular immune response inducing or enhancing agent comprising, as an active component, the nucleic acid structure according to any one of [1] to [8].
[30] The agent according to any one of [26] to [29], wherein the agent preferably comprises from 0.00001 to 100 mass% of the nucleic acid structure.
[31] Use of the nucleic acid structure according to any one of [1] to [8], for producing an allergen-specific IFNγ production enhancing agent.
[32] Use of the nucleic acid structure according to any one of [1] to [8], for producing an allergen-specific IgG2a production enhancing agent.
[33] Use of the nucleic acid structure according to any one of [1] to [8], for producing an allergen-specific Th1-type immune response inducing or enhancing agent.
[34] Use of the nucleic acid structure according to any one of [1] to [8], for producing an allergen-specific cellular immune response inducing or enhancing agent.
[35] The agent according to any one of [31] to [34], wherein the agent preferably comprises from 0.00001 to 100 mass% of the nucleic acid structure.
[36] Use of the nucleic acid structure according to any one of [1] to [8], for enhancing allergen-specific IFNγ production.
[37] Use of the nucleic acid structure according to any one of [1] to [8], for enhancing allergen-specific IgG2a production.
[38] Use of the nucleic acid structure according to any one of [1] to [8], for inducing or enhancing an allergen-specific Th1-type immune response.
[39] Use of the nucleic acid structure according to any one of [1] to [8], for inducing or enhancing an allergen-specific cellular immune response.
[40] The nucleic acid structure according to any one of [1] to [8], for use in enhancement of allergen-specific IFNγ production.
[41] The nucleic acid structure according to any one of [1] to [8], for use in enhancement of allergen-specific IgG2a production.
[42] The nucleic acid structure according to any one of [1] to [8], for use in induction or enhancement of an allergen-specific Th1-type immune response.
[43] The nucleic acid structure according to any one of [1] to [8], for use in induction or enhancement of an allergen-specific cellular immune response.
[44] A method for enhancing allergen-specific IFNγ production, the method comprising administering an effective amount of the nucleic acid structure according to any one of [1] to [8] to a subject in need thereof.
[45] A method for enhancing allergen-specific IgG2a production, the method comprising administering an effective amount of the nucleic acid structure according to any one of [1] to [8] to a subject in need thereof.
[46] A method for inducing or enhancing an allergen-specific Th1-type immune response, the method comprising administering an effective amount of the nucleic acid structure according to any one of [1] to [8] to a subject in need thereof.
[47] A method for inducing or enhancing an allergen-specific cellular immune response, the method comprising administering an effective amount of the nucleic acid structure according to any one of [1] to [8] to a subject in need thereof.
[48] The method according to any one of [44] to [47], wherein a dosage amount of the nucleic acid structure is preferably from 1 ng to 10 mg/60 kg of body weight a day, and preferably from 10 to 1 × 10¹⁵ virus particles/60 kg of body weight a day when the nucleic acid structure is a virus vector.
[49] The nucleic acid vaccine is administered orally or parenterally, preferably parenterally, in [10] to [17] and [21] to [25].
[50] The subject is an allergy patient or a human suspected to have an allergy in [20], [23] to [25] and [44] to [48].

### Examples

Hereinafter, the present invention will be described in further detail on the basis of Examples, which should not be construed as limiting the present invention.

### Example 1: Studies on SNARE protein

Messenger RNA (mRNA) was prepared in which each of the amino acid sequences (SEQ ID NOS: 25 to 45) of fusion polypeptides obtained by linking ovalbumin (OVA, amino acid sequence: SEQ ID NO: 24) as an allergen to the respective C-terminal sides of 21 types of human SNARE proteins by a linker (nucleotide sequence: SEQ ID NO: 19, amino acid sequence: SEQ ID NO: 20), or the amino acid sequence of only OVA was encoded. Here, a nucleotide sequence encoding the antigen or the fusion polypeptide was codon-optimized, and then incorporated into plasmid DNA (pVAX1 vector, Thermo Fisher Scientific). The nucleotide sequence of OVA incorporated into plasmid DNA is set forth as SEQ ID NO: 95, and the nucleotide sequences of the fusion polypeptides are set forth as SEQ ID NOS: 48 to 68. The nucleotide sequence of OVA before codon optimization is set forth as SEQ ID NO: 23. For preparation of mRNA, HiScribe T7 ARCA mRNA Kit (New England Biolabs Inc.) was used. Specifically, using a restriction enzyme, linearization was performed on plasmid DNA into which each sequence had been incorporated, and *in vitro* mRNA synthesis with T7 RNA polymerase and capping of the 5'-end with ARCA were then performed using the DNA as a template. Subsequently, DNase was added to degrade the DNA used as a template, followed by addition of a poly A chain by poly (A) polymerase. The obtained mRNA was purified with RNeasy Kit (QIAGEN N.V), and used for subsequent operations. The purified mRNA was transfected into matured human CD14⁺ dendric cells (Lonza) using TransIT-mRNA Transfection Reagent (Mirus Bio LLC.) in accordance with the accompanying protocol. As a control, a transfection reagent free from mRNA was added to dendric cells. From the day following the transfection, 1.0 × 10⁶ human CD4⁺ T cells (Lonza) and 2.5 × 10⁵ transfected dendric cells derived from the same donor were co-cultured for 7 days in total, and antigen presentation of mRNA-derived OVA was performed. Thereafter, the 1.0 × 10⁶ CD4⁺ T cells co-cultured on an ELISPOT plate were co-cultured again with 1.0 × 10⁵ human CD14⁺ dendric cells derived from the same donor, and 200 µg/mL of OVA protein was added to perform antigen stimulation. After 24 hours, CD4⁺ T cells producing IFNγ were detected by performing an ELISPOT assay using an ELISPOT assay kit (Cellular Technology Limited) in accordance with the accompanying protocol.

The results are shown in Fig. 1. Expression of a fusion protein of a specific SNARE protein (VAMP7, GOSR2, STX10, STX18, BNIP1, STX7, VTI1A, STX16, STX5, GOSR1, STX8, STX12, VAMP8 or SEC22B) and OVA in dendric cells led to a significant increase in the number of CD4⁺ T cells producing IFNγ in an OVA-specific manner. In general, while CD4⁺ T cells are differentiated into various cell species such as Th1, Th2, Th17 and Treg to release a specific cytokine when receiving antigen presentation, IFNγ is a cytokine which is characteristically secreted in Th1 cells. From these points, it is shown that by introducing a specific SNARE-OVA fusion protein according to the present invention into antigen-presenting cells such as dendric cells, a Th1 immune response can be induced or enhanced. The present experiment was conducted with n = 3 for each group. A group evaluated as meeting p < 0.05 when the Dunnett's test was conducted where a control group (Empty) subjected to restimulation with the OVA protein was used as a control was determined as giving a significant difference. A group evaluated as meeting p < 0.1 when the student's t-test was conducted using Empty as a control was also determined as giving a significant difference. The Empty (N.T.) group is a control group (Empty) which was not subjected to restimulation with the OVA protein.

### Example 2: IgG, IgG1 and IgG2a antibody titers

From the SNARE proteins that increased in production of IFNγ in Example 1, VAMP7 was selected, and a plasmid vector (pVAX1 vector, Thermo Fisher Scientific) was prepared in which the amino acid sequence (SEQ ID NO: 25) of a fusion polypeptide obtained by linking OVA to the C-terminal side of VAMP7 by a linker, or the amino acid sequence (SEQ ID NO: 24) of only OVA was encoded. As a control, an Empty vector was used in which the allergen (OVA) was not encoded. Each of the prepared vectors was intramuscularly administered in an amount of 50 µg into the femoral muscle of a female BALB/c mouse, and a voltage was then applied with an electroporator (NEPA21, Nepa Gene Co., Ltd.) to introduce a gene. On the 0th, 7th and 14th days after the start of the experiment, the treatment was performed, and on the 35th day, the mouse was euthanized, and the blood was collected. The serum was separated from the collected blood, and the antibody titer was measured. In the measurement of the antibody titer, a DPBS solution containing 10 µg/mL of OVA (Merck KGaA) was added to a 96-well ELISA plate (IWAKI) at 100 µL/well to immobilize the OVA. On the following day, the plate was washed, and then blocked with 100 µL/well of 1% BSA-containing DPBS for 1 hour. Subsequently, the plate was washed, and the separated serum was then added at 100 µL/well while being serially diluted by a factor of 2. After 2 hours, the serum was washed off, 1 µg/mL of an anti-mouse IgG, IgG1 or IgG2a antibody (Abeam plc) was added at 100 µL/well, followed by standing for 1 hour. Thereafter, a TMB solution (Abeam plc) was added at 50 µL/well to carry out a chromogenic reaction. After 10 minutes, a stop solution (Abeam plc) was added at 50 µL/well to stop the chromogenic reaction, and absorbance measurement (450 nm) was performed. Here, the value of a maximum dilution factor at which an absorbance value equal to or greater than twice the absorbance of a control containing DPBS instead of serum was obtained was defined as an antibody titer of the relevant sample.

The results are shown in Fig. 2. There was a significant decrease in OVA-specific IgG1 antibody titer in the VAMP7-OVA (V7-OVA) group as compared to the OVA-alone group. On the other hand, for OVA-specific IgG2a, there was a significant increase in antibody titer only in the VAMP7-OVA group as compared to the control group (Empty). For the antibody titer of OVA-specific IgG as a whole, there was no significant difference between the OVA-alone group and the VAMP7-OVA group. In general, it is known that while an IgG2a antibody is produced from B cells by a Th1-type immune response, an IgG1 antibody is produced from B cells by a Th2-type immune response. Thus, by measuring the antibody titers of allergen-specific IgG1 and IgG2a antibodies, the Th1/Th2 balance of the immune response caused in a living organism can be predicted. The present results show that VAMP7-OVA can also induce or enhance a Th1-type immune response *in vivo* because the OVA-specific IgG1 antibody titer decreased and the IgG2a antibody titer increased in VAMP7-OVA as compared to OVA alone. The present experiment was conducted with n = 10 for each group. If a group was evaluated as meeting p < 0.05 when the Tukey test was conducted, the group was determined as giving a significant difference.

### Example 3: ELISPOT assay

A plasmid vector (pVAX1 vector, Thermo Fisher Scientific) was prepared in which the amino acid sequence of a fusion polypeptide obtained by linking OVA to the C-terminal side of VAMP7 by a linker (V7-OVA), or the amino acid sequence of only OVA was encoded. As a control, an Empty vector was used in which the allergen (OVA) was not encoded. Each of the prepared vectors was intramuscularly administered in an amount of 50 µg into the femoral muscle of a female BALB/c mouse, and a voltage was then applied with an electroporator (NEPA21) to introduce a gene. On the 0th, 7th and 14th days after the start of the experiment, the above-described treatment was performed, and on the 35th day, the mouse was euthanized, and the spleen was extracted. The extracted spleen was ground on a 40 µm cell strainer while 2% FBS-containing DPBS was added. The obtained suspension liquid was centrifuged at 200×g for 5 minutes, the supernatant was removed, a Pharm Lyse^{™} solution (BD Bioscience) was then added, and the mixture was left standing for 3 minutes to dissolve erythrocytes contained in the suspension liquid. The liquid was centrifuged again at 200×g for 5 minutes, the supernatant was removed, washing was then performed twice with DPBS, and CTL-Test^{™} Medium (Cellular Technology Limited) was added to obtain a splenocyte suspension liquid. Splenocytes at 1.0 × 10⁷ cells/mL were seeded on an ELISPOT plate at 100 µL/well, and mixed with 100 µL of a CTL-test medium containing an OVA protein (Merck KGaA), thereby performing antigen stimulation at a final concentration of 50 µg/mL. As a negative control, a group free from an OVA protein (N. T) was provided. After 24 hours, cells producing IFNγ and IL-4 in an allergen (OVA)-specific manner were detected by performing an ELISPOT assay using an ELISPOT assay kit (Cellular Technology Limited) in accordance with the accompanying protocol. Since the spleen is a secondary lymphoid tissue, and splenocytes which are its constituent cells are abundant in T cells, splenocytes were used in the present experiment for detecting T cells which exhibit an allergen-specific immune response.

The results are shown in Fig. 3. When VAMP7-OVA (V7-OVA) was used, the number of T cells secreting IFNγ in an OVA-specific manner significantly increased while there was no difference in the number of T cells secreting IL-4 in an OVA-specific manner as compared to OVA alone. In general, it is known that IL-4 is secreted by Th2 cells, and IFNγ is secreted by Th1 cells, or cytotoxic T cells involved in cellular immunity (CD8⁺ T cells). Therefore, the present results show that by fusing VAMP7 with an allergen, an allergen-specific Th1-type immune response and cellular immune response can be selectively induced or enhanced without causing an allergen-specific Th2-type immune response. The present experiment was conducted with n = 10 for each group. If a group was evaluated as meeting p < 0.05 when the Tukey test was conducted under a non-stimulated condition (N. T) or an OVA-stimulated condition (OVA), the group was determined as giving a significant difference.

### Example 4: IgG, IgG1 and IgG2a antibody titers

A plasmid vector (pVAX1 vector, Thermo Fisher Scientific) was prepared in which the amino acid sequence (SEQ ID NO: 25, 27, 28 or 41) of a fusion polypeptide obtained by linking OVA to the C-terminal side of VAMP7, STX10, STX18 or GOSR1 by a linker (V7-OVA, STX10-OVA, STX18-OVA or GOSR1-OVA), or the amino acid sequence (SEQ ID NO: 24) of only OVA was encoded. As a control, an Empty vector was used in which the allergen (OVA) was not encoded. Each of the prepared vectors was intramuscularly administered in an amount of 50 µg into the femoral muscle of a female BALB/c mouse, and a voltage was then applied with an electroporator (NEPA21) to introduce a gene. On the 0th, 7th and 14th days after the start of the experiment, the above-described treatment was performed, and on the 35th day, the mouse was euthanized, and the blood was collected. The serum was separated from the collected blood, and the antibody titer was measured. In the measurement of the antibody titer, a DPBS solution containing 10 µg/mL of OVA (Merck KGaA) was added to a 96-well ELISA plate (IWAKI) at 100 µL/well to immobilize the OVA. On the following day, the plate was washed, and then blocked with 100 µL/well of 1% BSA-containing DPBS for 1 hour. Subsequently, the plate was washed, and the separated serum was then added at 100 µL/well while being serially diluted by a factor of 2. After 2 hours, the serum was washed off, 1 µg/mL of an anti-mouse IgG, IgG1 or IgG2a antibody (Abcam plc) was added at 100 µL/well, followed by standing for 1 hour. Thereafter, a TMB solution (Abcam plc) was added at 50 µL/well to carry out a chromogenic reaction. After 10 minutes, a stop solution (Abcam plc) was added at 50 µL/well to stop the chromogenic reaction, and absorbance measurement (450 nm) was performed. Here, the value of a maximum dilution factor at which an absorbance value equal to or greater than twice the absorbance of a control containing DPBS instead of serum was obtained was defined as an antibody titer of the relevant sample.

The results are shown in Fig. 4. In the fifth week after the administration of the vector, there was a significant increase in OVA-specific IgG antibody titer in the OVA-alone group and all of the groups with the respective SNARE sequences linked to OVA as compared to the Empty vector administration group. In particular, there was no change in OVA-specific IgG antibody titer in the VAMP7-OVA (V7-OVA) group and the GOSR1-OVA group as compared to the OVA-alone group, whereas there was a significant decrease in OVA-specific IgG antibody titer in the STX10-OVA group and the STX18-OVA group as compared to the OVA-alone group. The OVA-specific IgG1 antibody titer significantly decreased in all of the groups with the respective SNARE sequences linked to OVA as compared to the OVA-alone group. The OVA-specific IgG2 antibody titer significantly increased only in the V7-OVA group as compared to the Empty vector administration group. The IgG2a/IgG1 calculated as a ratio between the OVA-specific IgG1 and IgG2a antibody titers in each individual increased in all of the groups with the respective SNARE sequences linked to OVA as compared to the OVA-alone group although there was no significant difference. In the graph of IgG2a/IgG1, an average value is shown above the dotted plot for each group. The present results show that even in SNARE family proteins other than VAMP7, such as STX10, STX18 and GOSR1, the OVA-specific IgG1 antibody titer decreases and the IgG2a/IgG1 ratio increases, so that when the sequences of these SNARE proteins are linked to an allergen, an allergen-specific Th1-type immune response can also be induced or enhanced *in vivo.* The present experiment was conducted with n = 5 for each group. If a group was evaluated as meeting p < 0.05 when the Dunnett's test was conducted, the group was determined as giving a significant difference. A significant difference is indicated by * in the case of comparison with the Empty group, and by † in the case of comparison with the OVA group.

### Example 5: ELISPOT assay

A plasmid vector (pVAX1 vector, Thermo Fisher Scientific) was prepared in which the amino acid sequence (SEQ ID NO: 25, 27, 28 or 41) of a fusion polypeptide obtained by linking OVA to the C-terminal side of VAMP7, STX10, STX18 or GOSR1 by a linker (V7-OVA, STX10-OVA, STX18-OVA or GOSR1-OVA), or the amino acid sequence (SEQ ID NO: 24) of only OVA was encoded. As a control, an Empty vector was used in which the allergen (OVA) was not encoded. Each of the prepared vectors was intramuscularly administered in an amount of 50 µg into the femoral muscle of a female BALB/c mouse, and a voltage was then applied with an electroporator (NEPA21) to introduce a gene. On the 0th, 7th and 14th days after the start of the experiment, the above-described treatment was performed, and on the 35th day, the mouse was euthanized, and the spleen was extracted. The extracted spleen was ground on a 40 µm cell strainer while 2% FBS-containing DPBS was added. The obtained suspension liquid was centrifuged at 200×g for 5 minutes, the supernatant was removed, a Pharm Lyse solution (BD Bioscience) was then added, and the mixture was left standing for 3 minutes to lyse erythrocytes contained in the suspension liquid. The liquid was centrifuged again at 200×g for 5 minutes, the supernatant was removed, washing was then performed twice with DPBS, and CTL-Test Medium (Cellular Technology Limited) was added to obtain a splenocyte suspension liquid. On an ELISPOT plate, 1.0 × 10⁷ splenocytes were seeded at 100 µL/well, and mixed with 100 µL of a CTL-test medium containing an OVA protein (Merck KGaA), thereby performing antigen stimulation at a final concentration of 50 µg/mL. As a negative control, a group free from an OVA protein (N. T) was provided. After 24 hours, cells producing IFNγ and IL-4 in an allergen (OVA)-specific manner were detected by performing an ELISPOT assay using an ELISPOT assay kit (Cellular Technology Limited) in accordance with the accompanying protocol. Since the spleen is a secondary lymphoid tissue, and splenocytes which are its constituent cells are abundant in T cells, splenocytes were used in the present experiment for detecting T cells which exhibit an allergen-specific immune response.

The results are shown in Fig. 5. In all of the groups with the respective SNARE sequences linked to OVA, the number of T cells secreting IFNγ in an OVA-specific manner significantly increased as compared to the OVA-alone group. On the other hand, for the number of T cells secreting OVA-specific IL-4, there was a significant difference between the groups under each of a non-stimulated condition (N. T) and an OVA-stimulated condition (OVA), but for the IL-4 ratio calculated as a ratio in each individual under each of the N. T and OVA conditions, there was no change in any of the groups. The present results show that even in SNARE family proteins other than VAMP7, such as STX10, STX18 and GOSR1, linkage to an allergen enables an allergen-specific Th1-type immune response and cellular immune response to be selectively induced or enhanced without causing an allergen-specific Th2-type immune response. The present experiment was conducted with n = 5 for each group. If a group was evaluated as meeting p <0.05 when the Dunnett's test was conducted under a non-stimulated condition or an OVA-stimulated condition, the group was determined as giving a significant difference. A significant difference is indicated by * in the case of comparison with the Empty group, and by † in the case of comparison with the OVA group.

### Example 6: Antibody titer over time

A plasmid vector was prepared in which the amino acid sequence (SEQ ID NO: 25) of a fusion polypeptide obtained by linking OVA to the C-terminal side of VAMP7 by a linker (V7-OVA), the amino acid sequence (SEQ ID NO: 46) of a fusion polypeptide obtained by adding a proprotein convertase recognition sequence (nucleotide sequence: SEQ ID NO: 21, amino acid sequence: SEQ ID NO: 22) to the C-terminal side of the linker in the fusion polypeptide (V7-pc-OVA), the amino acid sequence (SEQ ID NO: 47) of a fusion polypeptide obtained by inserting the amino acid sequence of OVA into a lysosome-associated membrane protein LAMP (Patent Literature 1) (LAMP [OVA]), or the amino acid sequence (SEQ ID NO: 24) of only OVA was encoded. The nucleotide sequence of V7-pc-OVA incorporated into the plasmid DNA is set forth as SEQ ID NO: 69, and the nucleotide sequence of LAMP [OVA] is set forth as SEQ ID NO: 70. As a control, an Empty vector was used in which the allergen (OVA) was not encoded. Each of the prepared vectors was intramuscularly administered in an amount of 50 µg into the femoral muscle of a female BALB/c mouse, and a voltage was then applied with an electroporator (NEPA21) to introduce a gene. On the 0th, 7th and 14th days after the start of the experiment, the above-described treatment was performed. During the experiment, the blood was collected from the tail vein every week. On the 35th day, the mouse was euthanized, and the blood was collected. The serum was separated from the collected blood, and the antibody titer was measured. In the measurement of the antibody titer, a DPBS solution containing 10 µg/mL of OVA (Merck KGaA) was added to a 96-well ELISA plate (IWAKI) at 100 µL/well to immobilize the OVA. On the following day, the plate was washed, and then blocked with 100 µL/well of 1% BSA-containing DPBS for 1 hour. Subsequently, the plate was washed, and the separated serum was then added at 100 µL/well while being serially diluted by a factor of 2. After 2 hours, the serum was washed off, 1 µg/mL of an anti-mouse IgG, IgG1 or IgG2a antibody (Abcam plc) was added at 100 µL/well, followed by standing for 1 hour. Thereafter, a TMB solution (Abcam plc) was added at 50 µL/well to carry out a chromogenic reaction. After 10 minutes, a stop solution (Abcam plc) was added at 50 µL/well to stop the chromogenic reaction, and absorbance measurement (450 nm) was performed. Here, the value of a maximum dilution factor at which an absorbance value equal to or greater than twice the absorbance of a control containing DPBS instead of serum was obtained was defined as an antibody titer of the relevant sample.

The results are shown in Fig. 6. As in Example 2, there was a significant decrease in OVA-specific IgG1 antibody titer in the VAMP7-OVA (V7-OVA) group and the VAMP7-pc-OVA (V7-pc-OVA) group as compared to the OVA-alone group. On the other hand, for the OVA-specific IgG2a antibody titer, there was a more increase in antibody titer in the VAMP7-pc-OVA group. For the OVA-specific IgG as a whole, the antibody titer tended to increase more in the third and fourth weeks in the OVA-alone group, but in the fifth week, there was an equivalent increase in antibody titer particularly in the VAMP7-pc-OVA group as compared to the OVA-alone group. There was little increase in OVA-specific antibody titer in the LAMP [OVA] group. The IgG2a/IgG1 calculated as a ratio between OVA-specific IgG1 and IgG2a antibody titers in each individual in the fifth week increased in the VAMP7-OVA group and the VAMP7-pc-OVA group as compared to the OVA-alone group and the LAMP [OVA] group although there was no significant difference. The present results show that by introducing a proprotein convertase recognition sequence, production of the IgG2a antibody is further promoted, and an allergen-specific Th1-type immune response is induced or enhanced *in vivo.* It is also shown that in the VAMP7-OVA group and the VAMP7-pc-OVA group, allergen-specific IgG antibody production is improved and the allergen-specific Th1-type immune response is enhanced as compared to the LAMP [OVA] group. The present experiment was conducted with n = 5 for each group. If a group was evaluated as meeting p <0.05 when the Tukey test was conducted in each week, the group was determined as giving a significant difference. A significant difference is indicated by * in the case of comparison with the Empty group, and by † in the case of comparison with the OVA group.

### Example 7: ELISPOT assay

A plasmid vector was prepared in which the amino acid sequence (SEQ ID NO: 25) of a fusion polypeptide (V7-OVA) obtained by linking OVA to the C-terminal side of VAMP7 by a linker (V7-OVA), the amino acid sequence (SEQ ID NO: 46) of a fusion polypeptide obtained by adding a proprotein convertase recognition sequence to the C-terminal side of the liker in the fusion polypeptide (V7-pc-OVA), or the amino acid sequence (SEQ ID NO: 24) of only OVA was encoded. As a control, an Empty vector was used in which the allergen (OVA) was not encoded. Each of the prepared vectors was intramuscularly administered in an amount of 50 µg into the femoral muscle of a female BALB/c mouse, and a voltage was then applied with an electroporator (NEPA21) to introduce a gene. On the 0th, 7th and 14th days after the start of the experiment, the above-described treatment was performed, and on the 35th day, the mouse was euthanized, and the spleen was extracted. The extracted spleen was ground on a 40 µm cell strainer while 2% FBS-containing DPBS was added. The obtained suspension liquid was centrifuged at 200×g for 5 minutes, the supernatant was removed, a Pharm Lyse solution (BD Bioscience) was then added, and the mixture was left standing for 3 minutes to lyse erythrocytes contained in the suspension liquid. The liquid was centrifuged again at 200×g for 5 minutes, the supernatant was removed, washing was then performed twice with DPBS, and CTL-Test Medium (Cellular Technology Limited) was added to obtain a splenocyte suspension liquid. On an ELISPOT plate, 1.0 × 10⁷ splenocytes were seeded at 100 µL/well, and mixed with 100 µL of a CTL-test medium containing an OVA protein (Merck KGaA), thereby performing antigen stimulation at a final concentration of 50 µg/mL. As a negative control, a group free from an OVA protein (N. T) was provided. After 24 hours, cells producing IFNγ and IL-4 in an allergen (OVA)-specific manner were detected by performing an ELISPOT assay using an ELISPOT assay kit (Cellular Technology Limited) in accordance with the accompanying protocol. Since the spleen is a secondary lymphoid tissue, and splenocytes which are its constituent cells are abundant in T cells, splenocytes were used in the present experiment for detecting T cells which exhibit an allergen-specific immune response.

The results are shown in Fig. 7. Introduction of the proprotein convertase recognition sequence led to a further increase in the number of T cells secreting IFNγ in an OVA-specific manner. On the other hand, for the number of T cells secreting OVA-specific IL-4, there was a significant difference between the groups under each of a non-stimulated condition (N. T) and an OVA-stimulated condition (OVA), but for the IL-4 ratio calculated as a ratio in each individual under each of the N. T and OVA conditions, there was no change in any of the groups. The present results indicate that introduction of the proprotein convertase recognition sequences enables further enhancement of an allergen-specific Th1-type immune response and cellular immune response. The proprotein convertase is a type of protease, and plays a role in cleaving a precursor/immature protein to perform conversion into a mature/active protein in cells. Since the proprotein convertase is localized in golgi bodies and endosomes, it is considered that the VAMP7-pc-OVA fusion protein transported to intracellular vesicles was cleaved in a living organism to release OVA into the vesicles, so that association with MHC molecules was more efficiently induced. In addition, the released OVA may have been encapsulated in exosomes and secreted to induce enhancement of an immune response. The present experiment was conducted with n = 5 for each group. If a group was evaluated as meeting p <0.05 when the Tukey test was conducted under a non-stimulated condition (N. T) or an OVA-stimulated condition, the group was determined as giving a significant difference.

### Example 8: ELISPOT assay

A plasmid vector was prepared in which the amino acid sequence (SEQ ID NO: 25) of a fusion polypeptide obtained by linking OVA to the C-terminal side of VAMP7 by a linker (V7-OVA), the amino acid sequence (SEQ ID NO: 46) of a fusion polypeptide obtained by adding a proprotein convertase recognition sequence to the C-terminal side of the linker in the fusion polypeptide (V7-pc-OVA), the amino acid sequence (SEQ ID NO: 47) of a fusion polypeptide obtained by inserting the amino acid sequence of OVA into a lysosome-bound protein LAMP (Patent Literature 1) (LAMP [OVA]), or the amino acid sequence (SEQ ID NO: 24) of only OVA was encoded. As a control, an Empty vector was used in which the allergen (OVA) was not encoded. Each of the prepared vectors was intramuscularly administered in an amount of 50 µg into the femoral muscle of a female BALB/c mouse, and a voltage was then applied with an electroporator (NEPA21) to introduce a gene. On the 7th day after the treatment, the mouse was euthanized, and the spleen was extracted. The extracted spleen was ground on a 40 µm cell strainer while 2% FBS-containing DPBS was added. The obtained suspension liquid was centrifuged at 200×g for 5 minutes, the supernatant was removed, a Pharm Lyse solution (BD Bioscience) was then added, and the mixture was left standing for 3 minutes to lyse erythrocytes contained in the suspension liquid. The liquid was centrifuged again at 200×g for 5 minutes, the supernatant was removed, washing was then performed twice with DPBS, and CTL-Test Medium (Cellular Technology Limited) was added to obtain a splenocyte suspension liquid. On an ELISPOT plate, 1.0 × 10⁷ splenocytes were seeded at 100 µL/well, and mixed with 100 µL of a CTL-test medium containing an OVA protein (Merck KGaA), thereby performing antigen stimulation at a final concentration of 50 µg/mL. As a negative control, a group free from an OVA protein (N. T) was provided. After 24 hours, cells producing IFNγ and IL-4 in an allergen (OVA)-specific manner were detected by performing an ELISPOT assay using an ELISPOT assay kit (Cellular Technology Limited) in accordance with the accompanying protocol. Since the spleen is a secondary lymphoid tissue, and splenocytes which are its constituent cells are abundant in T cells, splenocytes were used in the present experiment for detecting T cells which exhibit an allergen-specific immune response.

The results are shown in Fig. 8. It was confirmed that even in just the first week after the administration of the vector, the number of T cells secreting IFNγ in an OVA-specific manner significantly increased in the VAMP7-pc-OVA group as compared to the VAMP7-OVA group or the LAMP [OVA] group. On the other hand, for the number of T cells secreting OVA-specific IL-4, there was a significant difference between the groups under each of a non-stimulated condition (N. T) and an OVA-stimulated condition (OVA), but for the IL-4 ratio calculated as a ratio in each individual under each of the N. T and OVA conditions, there was no change in any of the groups. Further, the ratio between the number of T cells producing IFNγ and the number of T cells producing IL-4, IFNy/IL-4, in each individual was calculated, and the result showed that during OVA stimulation, the IFNγ/IL-4 significantly increased in the V7-pc-OVA group as compared to the V7-OVA group and the LAMP [OVA] group. The present results show that in a relatively early period, i.e., in the first week, VAMP7-pc-OVA can selectively induce allergen-specific Th1-type immunity or cellular immunity without causing an allergen-specific Th2-type immune response, and has a superior immunity inducing effect as compared to conventional techniques. The present experiment was conducted with n = 5 for each group. If a group was evaluated as meeting p < 0.05 when the Tukey test was conducted under a non-stimulated condition (N. T) or an OVA-stimulated condition, the group was determined as giving a significant difference.

### Example 9: OVA food allergy model effectiveness

A sensitizing mixture was prepared by mixing 100 µg of OVA and 1 mg of an Alum adjuvant (Thermo Fisher Scientific) with 200 µL of DPBS, and subjecting the resulting mixture to inversion mixing for 30 minutes. On the 0th and 14th days, the prepared sensitizing mixture was intraperitoneally administered to a female BALB/c mouse to perform sensitization. A plasmid vector was prepared in which the amino acid sequence (SEQ ID NO: 24) of only OVA, the amino acid sequence (SEQ ID NO: 46) of a fusion polypeptide obtained by linking OVA to the C-terminal side of VAMP7 by a linker and a proprotein convertase recognition sequence (V7-pc-OVA), or the amino acid sequence (SEQ ID NO: 47) of a fusion polypeptide obtained by inserting the amino acid sequence of OVA into LAMP (LAMP [OVA]) was encoded. As a control, an Empty vector (Empty) was provided in which the allergen (OVA) was not encoded. On the 21st, 28th and 35th days after the start of the experiment, 50 µg of each of the prepared vectors was intramuscularly administered into the femoral muscle of the BALB/c mouse, and a voltage was then applied with an electroporator (NEPA21) to introduce a gene. On the 42nd, 43rd, 44th, 45th and 46th days after the start of the experiment, a challenging mixture obtained by dissolving 50 mg of OVA in 200 µL of DPBS was orally administered once a day to induce a food allergy symptom. On the 46th day after the start of the experiment, the rectal temperature was measured using a body temperature measuring probe (Natsume Seisakusho Co., Ltd.) before administration of the challenging mixture and 15, 30, 45 and 60 minutes after the start of the administration. Thereafter, the mouse was euthanized, and the blood was collected. The serum was separated from the collected blood, and the OVA-specific IgE concentration was measured. LBIS Mouse anti-OVA-IgE ELISA Kit (FUJIFILM Wako Shibayagi Corporation) was used in the measurement of the OVA-specific IgE concentration. To a 96-well plate on which an OVA protein was immobilized, 50 µL of a biotin-bound anti-IgE antibody and 10 µL of diluted serum or a standard solution were added, and the mixture was left standing at room temperature for 1 hour to react. Subsequently, washing was performed three times with a wash solution, 100 µL of a peroxidase-avidin conjugate was then added, and the mixture was left standing at room temperature for 30 minutes to react. Subsequently, washing was performed three times, and 100 µL of a TMB chromogenic solution was then added to carry out a chromogenic reaction at room temperature. After 20 minutes, 100 µL of 1 M H₂SO₄ was added to stop the chromogenic reaction, and absorbance measurement was performed (dominant wavelength: 450 nm, sub-wavelength: 620 nm). The OVA-specific IgE concentration in each serum sample was calculated using a calibration curve obtained from the absorbance values of the standard solution.

The results are shown in Fig. 9. As compared to the OVA-non-sensitized Empty group (Empty (-)), the OVA-sensitized Empty group (Empty (+)), OVA-alone group (OVA (+)) and LAMP [OVA] group (LAMP [OVA] (+)) underwent a significant decrease in body temperature due to anaphylaxis in oral OVA sensitization. On the other hand, in the OVA-sensitized VAMP7-pc-OVA group (V7-pc-OVA (+)), there was not a decrease in body temperature as compared to the Empty (-) group, and a decrease in body temperature was significantly suppressed as compared to the Empty (+), OVA (+) and LAMP [OVA] (+) groups. The OVA-specific IgE antibody concentration significantly increased in the OVA (+) group as compared to the Empty (-) group, whereas the OVA-specific IgE antibody concentration significantly decreased in the V7-pc-OVA (+) group and the LAMP [OVA] group as compared to the OVA (+) group. Given the fact that a decrease in body temperature due to anaphylaxis is a general phenotype in an allergy animal model, and the allergen-specific IgE concentration in the blood is a general marker indicating a state of the allergy, these results show that VAMP7-pc-OVA has a superior allergy suppressing effect as compared to OVA alone and conventional techniques. The renal temperature measurement was performed with n = 8 for each group. If a group was evaluated as meeting p < 0.05 when the Dunnett's test was conducted at each measurement time, the group was determined as giving a significant difference. A significant difference is indicated by * in the case of comparison with the Empty (-) group, and by † in the case of comparison with the V7-pc-OVA (+) group. The OVA-specific IgE concentration measurement was performed with n = 7 for the Empty (+) group and with n = 8 for other groups. If a group was evaluated as meeting p < 0.05 when the Tukey test was conducted, the group was determined as giving a significant difference. For the V7-pc-OVA group and the LAMP [OVA] group, an average value is shown above the dotted plot for each group.

### Example 10: Blood concentration of OVA

A plasmid vector was prepared in which the amino acid sequence (SEQ ID NO: 25) of a fusion polypeptide obtained by linking VAMP7 and OVA by a linker (V7-OVA), the amino acid sequence (SEQ ID NO: 46) of a fusion polypeptide obtained by adding a proprotein convertase recognition sequence to the rear part of the linker in the fusion polypeptide (V7-pc-OVA), or the amino acid sequence (SEQ ID NO: 24) of only OVA was encoded. As a control, an Empty vector (Empty) was provided in which the allergen (OVA) was not encoded. Each of the provided vectors was intramuscularly administered in an amount of 50 µg into the femoral muscle of a female BALB/c mouse, and a voltage was then applied with an electroporator (NEPA21) to introduce a gene. On the 7th day after the above-described treatment, the mouse was euthanized, and the blood was collected. The serum was separated from the collected blood, and the OVA concentration was measured. ITEA Ovalbumin (OVA) ELISA Kit (ITEA Inc.) was used in the measurement of the OVA concentration. To a 96-well plate on which an anti-OVA antibody was immobilized, the serum or a standard solution was added at 100 **µ**L/well, and the mixture was left standing at room temperature for 1 hour to react. Subsequently, washing was performed three times with a wash solution, an enzyme-labeled anti-OVA antibody was then added at 100 **µ**L/well, and the mixture was left standing at room temperature for 1 hour to react. Subsequently, washing was performed three times, and a TMB chromogenic substrate solution was then added at 100 **µ**L/well to carry out a chromogenic reaction at room temperature. After 15 minutes, a reaction stopping solution was added at 100 **µ**L/well to stop the chromogenic reaction, and absorbance measurement was performed (dominant wavelength: 450 nm, sub-wavelength: 620 nm). The OVA concentration in each serum sample was calculated using a calibration curve obtained from the absorbance values of the standard solution.

The results are shown in Fig. 10. It was confirmed that in the OVA-alone group, an OVA protein was detected in the blood in the first week after the administration of the vector as compared to the Empty vector administration group (Empty). On the other hand, in the VAMP7-OVA (V7-OVA) group and the VAMP7-pc-OVA (V7-pc-OVA) group, the OVA protein concentration in the blood decreased to a level substantially equivalent to that in the Empty group as compared to the OVA-alone group. The present results indicate that in the case of administration as OVA alone *in vivo,* a part of the OVA protein expressed in a living organism is released into the blood. On the other hand, in the V7-OVA group and the V7-pc-OVA group, there may be little OVA protein in the blood in the first week because the blood concentration of an OVA protein decreases to a level equivalent in the Empty group where an OVA protein is not expressed in essence. It is known that in conventional allergen immunotherapies, a part of an exogenously ingested allergen is released into the blood, and caught by an allergen-specific IgE antibody, thus causing a side effect which falls under the category of type I allergies such as anaphylaxis. The present results indicate that when an allergen is linked to VAMP7, or VAMP7 and a proprotein convertase recognition sequence rather than being an allergen alone, it may be possible to suppress release of an allergen into the blood, leading to reduction of the therapy-associated side effect which falls under the category of type I allergies. The present experiment was conducted with n = 5 for each group. If a group was evaluated as meeting p <0.05 when the Tukey test was conducted, the group was determined as giving a significant difference.

## Claims

1. A nucleic acid structure comprising a polynucleotide encoding a SNARE protein and a polynucleotide encoding an allergen.

2. The nucleic acid structure according to claim 1, wherein the polynucleotide encoding an allergen is linked downstream of the polynucleotide encoding a SNARE protein.

3. The nucleic acid structure according to claim 1 or 2, wherein the polynucleotide encoding a SNARE protein and the polynucleotide encoding an allergen are connected via a polynucleotide encoding a linker and/or a polynucleotide encoding a proprotein convertase recognition sequence.

4. The nucleic acid structure according to any one of claims 1 to 3, which is a plasmid vector, mRNA or a virus vector.

5. The nucleic acid structure according to any one of claims 1 to 4, wherein the SNARE protein is any one selected from the group consisting of VAMP7, GOSR2, STX10, STX18, BNIP1, STX7, VTI1A, STX16, STX5, GOSR1, STX8, STX12, VAMP8 and SEC22B.

6. An allergen-specific Th1-type immune response inducing or enhancing agent comprising, as an active component, the nucleic acid structure according to any one of claims 1 to 5.

7. An allergen-specific cellular immune response inducing or enhancing agent comprising, as an active component, the nucleic acid structure according to any one of claims 1 to 5.

8. A nucleic acid vaccine comprising, as an active component, the nucleic acid structure according to any one of claims 1 to 5.

9. The nucleic acid vaccine according to claim 8, which induces or enhances an allergen-specific Th1-type immune response.

10. The nucleic acid vaccine according to claim 8, which induces or enhances an allergen-specific cellular immune response.

11. The nucleic acid vaccine according to any one of claims 8 to 10, which is vaccine for prevention or treatment of an allergy.

12. The nucleic acid vaccine according to any one of claims 8 to 11, which is administered orally or parenterally.
